# FASCICULE DE BREVET EUROPEEN

(11) **EP 0 728 842 B1**
(45) Date de publication et mention de la délivrance du brevet: **28.07.2004**
(21) Numéro de dépôt: 95402873.4
(22) Date de dépôt: 19.12.1995
(51) Int. Cl.: C12N 15/86, A61K 39/17, A61K 39/215, A61K 39/245, A61K 39/12

(54) **Vaccin vivant recombinant aviaire à base de virus herpès aviaire, notamment contre la maladie de gumboro**
Lebende rekombinante Vogelvakzine auf eines Vogelherpesvirus gegründet, gegen Gumborokrankheit
Live recombinant avian vaccine based on an avianherpes virus, against Gumboro disease

(30) Priorité: 30.12.1994 FR 9416015
(43) Date de publication de la demande: 28.08.1996
(73) Titulaire: MERIAL, 69002 Lyon (FR)
(72) Inventeur: Audonnet, Jean-Christophe Francis, F-69006 Lyon (FR); Bublot, Michel Joseph Marie, F-69290 Saint-Genis-Les-Ollieres (FR); Darteil, Raphael, Jean, F-91230 Montgeron (FR); Duinat, Carole Véronique, F-69007 Lyon (FR); Laplace, Eliane Louise Françoise, F-69600 Oullins (FR); Riviere, Michel Albert Emile, F-69130 Ecully (FR)
(74) Mandataire: Nargolwalla, Cyra

(56) Documents cités:
- EP-A- 0 477 056
- WO-A-93/25665
- WO-A-96/05291
- FR-A- 2 697 534
- VIROLOGY, vol. 195, 1993, pages 638-648, XP002017109 D. MARSHALL ET AL.: "Selection of Marek's disease virus recombinants expressing the E. coli gpt gene"
- J. GEN. VIROL., vol. 72, 1991, pages 897-906, XP002017110 C. MACLEAN ET AL.: "Investigation of herpes simplex virus type 1 genes encoding multiply inserted membrane proteins"
- VIROLOGY, vol. 199, 1994, pages 81-88, XP002017111 L. POWERS ET AL.: "Characterisation of the prv43 gene of pseudorabies virus and demonstraion that it is not required for growth in culture"

## Description

La présente invention a trait à des vaccins à usage aviaire à base de virus herpès aviaires recombinants vivants, à savoir notamment de virus de la maladie de Marek (MDV), et plus particulièrement de virus HVT (Herpes Virus of Turkey), dans lesquels a été insérée, par recombinaison génétique, au moins une séquence nucléotidique codant pour, et exprimant, un polypeptide antigénique d'un agent pathogène aviaire, dans des conditions assurant une immunisation conduisant à une protection efficace de l'animal vacciné contre ledit agent pathogène. Elle s'applique d'autre part au virus de la laryngotrachéite infectieuse (ILTV) et au virus de l'herpès du canard.

On a déjà proposé un certain nombre de vecteurs viraux aviaires recombinants dans l'intention de vacciner les oiseaux contre des agents pathogènes aviaires, notamment des virus pathogènes, parmi lesquels figurent les virus de la maladie de Marek (MDV), de la maladie de Newcastle (NDV), de la laryngotrachéite infectieuse (ILTV), de la maladie de Gumboro (Infectious Bursal Disease, IBDV), de la bronchite infectieuse (IBV) et de l'anémie aviaire (CAV).

Les vecteurs viraux utilisés comprennent des avipox, en particulier le Fowlpox (EP-A-0 517 292 ; H.-G. Heine *et al.,* Arch. Virol. 1993, **131**. 277-292 ; D.B. Boyle *et al*., Veterinary Microbiology 1994, **41**, 173-181 ; C.D. Bayliss *et al.,* Arch. Virol. 1991, **120**. 193-205), les virus de Marek, notamment les sérotypes 2 et 3 (HVT) (WO-A-87 04463 ; WO-A-89 01040 ; WO-A-93 25665 ; EP-A-0 513 921 ; J. McMillen, Poultry Condemnation Meeting, October 1994, 359-363 ; P.J.A. Sondermeijer *et al.,* Vaccine 1993, **11**. 349-357 ; R.W. Morgan *et al.,* Avian Diseases 1992. **36**. 858-870, et 1993. **37**. 1032-1040) ou encore le virus ILTV et l'adénovirus aviaire.

Lorsqu'ils sont utilisés pour la vaccination, ces virus recombinants induisent des protections de niveaux variables, généralement faibles ou partielles, même si, dans de rares cas particuliers, une protection importante peut être démontrée.

Parmi les protections les plus difficiles à assurer par des vaccins aviaires vivants recombinants, se trouve celle contre le virus de la maladie de Gumboro, ou virus IBDV. En effet, bien qu'il existe des vaccins vivants atténués ou inactivés classiques efficaces contre cette maladie, aucun vaccin vivant recombinant n'a encore fait preuve d'une efficacité convenable.

Le génome du virus de la maladie de Gumboro est constitué d'un ARN double brin. Le segment le plus grand (segment A) code pour une polyprotéine de 115 kDa secondairement clivée en trois protéines VP2 (41 kDa), VP4 (28 kDa), VP3 (32 kDa). VP4 serait une protéase intervenant dans la maturation de la polyprotéine 115 kDa. La position du site de clivage entre VP2 et VP4 n'est déterminée qu'approximativement (M. Jagadish, J. Virol. 1988, **62**. 1084-1087). La protéine VP2 est un immunogène induisant des anticorps neutralisants et une protection contre la maladie de Gumboro.

On a déjà proposé d'insérer des gènes codant pour des protéines immunogènes d'IBDV dans divers vecteurs vivants : EP-A-0 517 292 (insertion de séquences codant pour VP2 ou la polyprotéine dans un avipox) ; C.D. Bayliss 1991, H.-G. Heine 1993 et D.B. Boyle 1994 *supra* (VP2 dans le Fowlpox) ; WO-A-90 02802 (vecteur MDV) ; WO-A-90 02803 (vecteurs HVT et ILTV) ; demandes de brevet français n° 90 03105, 90 11146 et 92 13109 (vecteur HVT).

Les demandes de brevet WO-A-89 01040 et WO-A-93 25655 décrivent la construction d'un vecteur viral aviaire HVT dans lequel a été insérée, dans le site d'insertion Xhol présent dans le gène UL43 de HVT, identifié dans la première de ces deux demandes sous le nom de fragment BamHI #16, sous le contrôle d'un promoteur exogène, une séquence permettant d'exprimer la protéine VP2 native du virus IBDV, connue comme le meilleur candidat polypeptidique pour la vaccination contre la maladie de Gumboro. Cependant, malgré l'obtention d'une protéine native VP2, aucune induction d'anticorps neutralisants ni protection n'ont pu être obtenues lorsque les animaux vaccinés ont été éprouvés par le virus IBDV virulent.

C'est pourquoi l'idée d'utiliser ce site n'a pas été poursuivie et il a été proposé, à la place, dans WO-A-93 25655, d'insérer des séquences permettant d'exprimer la protéine VP2 d'IBDV, toujours sous le contrôle d'un promoteur exogène, dans le site unique Stul du gène US2 de HVT. L'expression de la protéine VP2 a été caractérisée *in vitro,* sans démonstration d'activité, ni de protection *in vivo.* On peut aussi noter que des insertions d'autres gènes dans ce site Stul de US2, à savoir le gène gB de MDV, accompagné ou non du gène gC (nommé gA dans ce document antérieur), et associé à son propre promoteur, ou les gènes gB et gC avec le gène F de NDV associé au promoteur HCMV immediate early (IE), ou les gènes gB et gC avec le gène HN de NDV associé au promoteur PRV gX, ont permis d'obtenir une protection contre les épreuves par les virus virulents correspondants. De même, une protection est signalée pour des constructions insérant, dans ce même site, les gènes gB et gD du virus ILTV. Les résultats contrastent avec l'absence totale de protection et les simples suppositions de la demande WO-A-89 01040.

Divers promoteurs, y compris ceux généralement disponibles dans le commerce, ont été utilisés dans les différentes constructions de l'art antérieur, parmi lesquels les promoteurs PRV gX, HCMV lE (CMV immediate early humain), herpes simplex alpha-4, FPV P.E/L (promoteur Fowlpox) (H. Heine *et al.,* Arch. Virol. 1993. **131**. 277-292), P7.5 (C. Bayliss *et al.,* Arch. Virol. 1991. **120**. 193-205) et P11 (D. Boyle *et al.,* Vet. Microb. 1994. **41**. 173-181) du virus de la vaccine, provenant de la séquence LTR du virus RSV (Rous Sarcoma Virus), SV40 précoce, ainsi que des promoteurs MDV ou HVT, tels que les promoteurs des gènes gB, gC, TK, RR2, etc., sans qu'ait pu apparaître une règle, notamment dans le cas des constructions dans HVT.

Les séquences de certains promoteurs peuvent inhiber la réplication des vecteurs recombinants HVT ou MDV (D.R. Marshall *et al.,* J. Vir. Meth. 1992. **40**. 195-204 et Virology 1993. **195**. 638-648). Parmi les promoteurs cités, un certain nombre, comme par exemple SV40, LTR RSV et PRV gX, ont montré une certaine efficacité, de même que certains promoteurs propres de certains gènes des virus Marek, notamment de sérotype 3.

Le besoin d'un vaccin vivant recombinant efficace contre le virus de la maladie de Gumboro (IBDV) existe donc toujours, en raison des contraintes techniques et économiques imposées par les vaccins inactivés et des problèmes d'innocuité liés à l'usage des vaccins vivants atténués. Un tel vaccin à base d'un vecteur HVT recombinant qui serait réellement efficace contre la maladie de Gumboro, pourrait en outre ouvrir la voie à des vaccins très efficaces contre d'autres maladies aviaires, la protection contre la maladie de Gumboro étant particulièrement difficile.

Or l'invention a permis, de façon surprenante, de mettre au point un vaccin vivant recombinant à base d'un vecteur HVT dans lequel est insérée au moins une séquence codant pour la protéine VP2 d'IBDV, qui assure une protection totale des animaux contre la maladie de Gumboro, à savoir protection vis-à-vis de la mortalité et des lésions de la bourse de Fabricius. Bien plus, l'efficacité de ce vaccin s'avère telle qu'il est devenu possible de vacciner efficacement et sans effets secondaires (notamment absence de lésions locales au point d'injection et absence de lésion de la bourse de Fabricius) même des poussins d'un jour, ce qui n'était même pas possible avec les vaccins inactivés. De plus, les doses nécessaires sont étonnamment faibles.

La présente invention a pour objet un vaccin vivant recombinant aviaire comprenant, comme vecteur, un virus herpès aviaire comprenant au moins une séquence nucléotidique codant pour, et exprimant, un polypeptide antigénique d'un agent pathogène aviaire, insérée dans le gène UL43 sous le contrôle du promoteur CMV immediate early.

Les virus herpès aviaires selon l'invention sont de préférence les virus de la maladie de Marek, notamment HVT, le virus de la laryngotrachéite infectieuse ILTV et l'herpès du canard. Les virus de la maladie de Marek et plus particulièrement le virus HVT sont préférés.

Par insertion dans le gène UL43, on entend aussi bien la simple insertion dans ce site sans délétion de celui-ci, que l'insertion après délétion totale ou partielle. On préfère l'insertion après délétion partielle.

Par promoteur CMV immediate early (IE), on entend le fragment donné dans les exemples ainsi que ses sous-fragments conservant la même activité promotrice. Le promoteur CMV lE peut être le promoteur humain (HCMV IE) ou le promoteur murin (MCMV IE), ou encore un promoteur CMV lE d'une autre origine, par exemple du rat ou du cochon d'Inde.

La séquence nucléotidique insérée dans le vecteur Marek pour être exprimée peut être toute séquence codant pour un polypeptide antigénique, d'un agent pathogène aviaire, capable, une fois exprimé dans les conditions favorables procurées par l'invention, d'assurer une immunisation conduisant à une protection efficace de l'animal vacciné contre l'agent pathogène. On pourra donc insérer, dans les conditions de l'invention, les séquences nucléotidiques codant pour les antigènes d'intérêt pour une maladie donnée. Les caractéristiques du recombinant selon l'invention permettent en particulier la vaccination *in ovo* et la vaccination des poussins d'1 jour, ainsi que celle des poussins plus âgés et des adultes.

Le cas typique de l'invention est l'insertion d'une séquence nucléotidique codant convenablement pour le polypeptide VP2 du virus IBDV. On obtient ainsi un vaccin vivant recombinant assurant, en plus d'une protection contre la maladie de Marek, une protection totale contre la maladie de Gumboro. Si on le souhaite, on peut aussi insérer une séquence codant pour un autre antigène d'IBDV, tel que VP3 ou encore la polyprotéine VP2 + VP4 + VP3, ces autres possibilités n'étant pas préférées.

Le vaccin recombinant contre la maladie de Gumboro se présentera de préférence sous de 10 à 10⁴ PFU/dose, plus particulièrement de 10² à 10³ PFU/dose et même entre 10 et 10² PFU/dose environ.

D'autres cas préférés de l'invention sont l'insertion de séquences nucléotidiques codant pour des antigènes du virus de la maladie de Marek, en particulier gènes gB, gC, gD et gH+gL (WO-A-90 02803), du virus de la maladie de Newcastle, en particulier gènes F et HN, du virus de la bronchite infectieuse (IBV), en particulier gènes S et M (M. Binns *et al.,* J. Gen. Virol. 1985. **66**. 719-726 ; M. Boursnell *et al.,* Virus Research 1984, **1**. 303-313), du virus de l'anémie aviaire (CAV), en particulier VP1 (52kDa) + VP2 (24kDa) (N.H.M. Noteborn *et al.,* J. Virol. 1991, **65**. 3131-3139) et du virus de la laryngotrachéite infectieuse (ILTV), en particulier gènes gB (WO-A-90 02802), gC, gD et gH + gL.

Les doses seront de préférence les mêmes que celles indiquées pour le vaccin Gumboro.

Selon un développement avantageux de l'invention, on associe au promoteur CMV lE un autre promoteur selon une disposition tête-bêche, ce qui permet d'insérer, dans la région d'insertion, deux séquences nucléotidiques, l'une sous la dépendance du promoteur CMV IE, l'autre sous celle du promoteur associé. Cette construction est remarquable par le fait que la présence du promoteur CMV IE, et notamment de sa partie activatrice (enhancer), active la transcription induite par le promoteur associé. Un promoteur associé préféré est le promoteur Marek RNA 1.8, dont l'activité de transcription s'est révélée multipliée par 4,4 environ dans ces conditions.

Le cas typique de l'invention est un vaccin comprenant une séquence nucléotidique codant pour VP2 d'IBDV sous le contrôle de CMV lE et une séquence nucléotidique codant pour un antigène d'une autre maladie aviaire, notamment celles citées plus haut, sous le contrôle de l'autre promoteur.

On peut aussi monter tête-bêche deux promoteurs CMV lE d'origines différentes.

Le promoteur RNA 1.8 pourra aussi être utilisé seul à la place du promoteur CMV IE, notamment pour des vaccins contre la maladie de Marek, la maladie de Newcastle, la laryngotrachéite infectieuse, la bronchite infectieuse et l'anémie aviaire.

La présente invention a aussi pour objet une formule de vaccin multivalent, comprenant, en mélange ou à mélanger, au moins deux vaccins vivants recombinants aviaires tels que définis plus haut, ces vaccins comprenant des séquences insérées différentes, notamment de pathogènes différents.

La présente invention a aussi pour objet une méthode de vaccination aviaire comprenant l'administration d'un vaccin vivant recombinant ou d'une formule de vaccin multivalent tel que défini plus haut. Elle a notamment pour objet une telle méthode pour la vaccination *in ovo,* pour la vaccination des poussins d'1 jour ou plus âgés, et pour la vaccination des adultes.

L'invention va être maintenant décrite plus en détail à l'aide d'exemples de réalisation non limitatifs, pris en référence au dessin, dans lequel :
**Figure 1** : Séquence de HVT U_{S} région gl
**Figure 2** : Construction des plasmides pRD022 et pRD027
**Figure 3** : Construction des plasmides pRD023 et pRD029
Figure **4** : plasmide pCMVβ
**Figure 5** : plasmide pEL022
**Figure 6** : plasmide pEL023
**Figure 7** : plasmide pEL024
**Figure 8** : plasmide pEL025
**Figure 9** : Séquence du fragment HVT BamHI M et ORF UL43
**Figure 10** : Construction des plasmides pMB010 et pMB016
**Figure 11** : plasmide pEL026
**Figure 12** : plasmide pEL027
**Figure 13** : plasmide pEL042
**Figure 14** : plasmide pCD002
**Figure 15** : plasmide pCD009
**Figure 16** : plasmide pEL068
**Figure 17** : plasmide pEL070
**Figure 18** : plasmide pEL072
**Figure 19** : plasmide pCD011
**Figure 20** : plasmide pCD012
**Figure 21** : Séquence du gène NDV HN
**Figure 22** : plasmide pEL028
**Figure 23** : plasmide pEL029bis
**Figure 24** : plasmide pEL030
**Figure 25** : plasmide pEL032
**Figure 26** : plasmide pEL043
**Figure 27** : plasmide pEL033
**Figure 28** : plasmide pEL034
**Figure 29** : plasmide pEL044
**Figure 30** : Séquence du promoteur RNA 1.8 kpb
**Figure 31** : plasmide pBS002
**Figure 32** : plasmide pEL069
**Figure 33** : plasmide pEL080
**Figure 34** : plasmide pEL081
**Figure 35** : plasmide pEL082
**Figure 36** : plasmide pEL096

Liste des séquences SEQ ID pour les constructions dans le site UL43
**SEQ ID N° 1** Oligonucléotide RD045
**SEQ ID N° 2** Oligonucléotide pBRPst-
**SEQ ID N° 3** Séquence HVT Uₛ région gl
**SEQ ID N° 4** Oligonucléotide RD048
**SEQ ID N° 5** Oligonucléotide RD049
**SEQ ID N° 6** Séquence fragment HVT BamHI M
**SEQ ID N° 7** Oligonucléotide MB014
**SEQ ID N° 8** Oligonucléotide MB015
**SEQ ID N° 9** Oligonucléotide MB070
**SEQ ID N° 10** Oligonucléotide MB071
**SEQ ID N° 11** Oligonucléotide CD001
**SEQ ID N° 12** Oligonucléotide CD002
**SEQ ID N° 13** Oligonucléotide CD003
**SEQ ID N° 14** Oligonucléotide CD004
**SEQ ID N° 15** Séquence du gène NDV HN
**SEQ ID N° 16** Oligonucléotide EL071
**SEQ ID N° 17** Oligonucléotide EL073
**SEQ ID N° 18** Oligonucléotide EL074
**SEQ ID N° 19** Oligonucléotide EL075
**SEQ ID N° 20** Oligonucléotide EL076
**SEQ ID N° 21** Oligonucléotide EL077
**SEQ ID N° 22** Séquence du promoteur RNA 1.8 kpb
**SEQ ID N° 23** Oligonucléotide MB047
**SEQ ID N° 24** Oligonucléotide MB048
**SEQ ID N° 25** Oligonucléotide MB072

### 2. EXEMPLES

Toutes les constructions de plasmides ont été réalisées en utilisant les techniques standards de biologie moléculaire décrites par Sambrook J. *et al.* (*Molecular Cloning: A Laboratory Manual.* 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).
Le virus utilisé comme virus parental est la souche FC126 de l'herpèsvirus de la dinde (HVT) isolé par le Dr. Witter du Regional Poultry Research Laboratory (USDA, East Lansing, Michigan), dans un troupeau de dindes de 23 semaines (Witter R.L. *et al.* Am. J. Vet. Res. 1970. **31**. 525-538). Les conditions de culture de ce virus sont celles décrites par ailleurs (demande de brevet français 90 03105).

### Exemple 1: Extraction de l'ADN du virus de la maladie de Marek:

Le sang total d'un poulet éprouvé à 7 jours avec la souche MDV RB1 B est récolté avec une seringue sur anticoagulant (solution d'héparine à 100 Ul/ml) 14 jours après infection. Ce sang est ensuite centrifugé à 30 g pendant 15 minutes à température ambiante. Le plasma ainsi que le "buffy-coat" sont prélevés et dilués dans du PBS stérile pour avoir un volume final de 10 ml. Après une centrifugation de 15 minutes à 150 g, le culot cellulaire est resuspendu dans 2 ml de milieu de culture 199 (Gibco-BRL Cat# 042-01183M) contenant 2 % de sérum de veau foetal (SVF)).
L'ADN total des lymphocytes infectés est ensuite extrait selon la technique décrite par R. Morgan *et al.* (Avian Diseases. 1990. **34**. 345-351) et peut directement servir de matrice pour les expériences de PCR. Pour le clonage de fragments génomiques du virus MDV, la souche RB1 B a été cultivée sur CEP et l'ADN viral a été préparé à partir de particules virales purifiées comme décrit par Lee Y. *et al.* (J. Gen. Virol. 1980. **51**. 245-253).

### Exemple 2: Préparation de l'ADN génomique du virus MCMV (Mouse CytoMegaloVirus)

Le virus MCMV souche Smith a été obtenu de l'American Type Culture Collection, Rockville, Maryland, USA (ATCC N° VR-194). Ce virus a été cultivé sur cellules d'embryon de souris Balb/C et l'ADN viral de ce virus a été préparé comme décrit par Ebeling A. *et al.* (J. Virol. 1983. **47**. 421-433).

### Exemple 3: Préparation de l'ADN génomique du virus HVT pour les expériences de transfection:

L'ADN viral utilisé pour les expériences de transfection a été préparé selon la technique décrite par R. Morgan *et al.* (Avian Diseases. 1990. **34**. 345-351) à partir d'une culture de CEP secondaires (CEP II) infectées avec la souche FC126 du virus HVT.

### Construction des virus recombinants

### Exemple 4: Construction et isolement de vHVT1

La construction du plasmide donneur pGH010, l'isolement et la purification du virus recombinant vHVT1 ont été décrits dans la demande de brevet français 92.13109. Ce virus HVT recombinant contient le gène codant pour la protéine de capside VP2 du virus de la maladie de Gumboro (virus "IBDV") placé sous le contrôle du promoteur du gène RR2 du virus HVT. Dans ce virus recombinant, le gène VP2 a été inséré à la place du gène HVT RR2.

### Exemple 5: Construction du plasmide donneur pEL025 et isolement de vHVT2

### 5.1. Construction du plasmide donneur pEL025

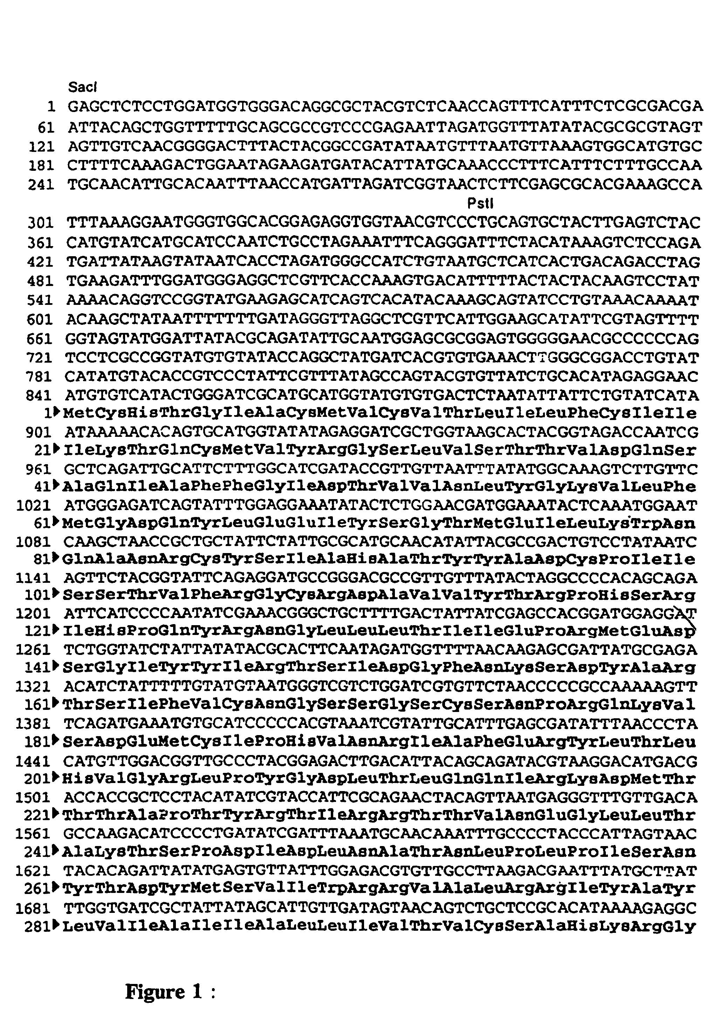

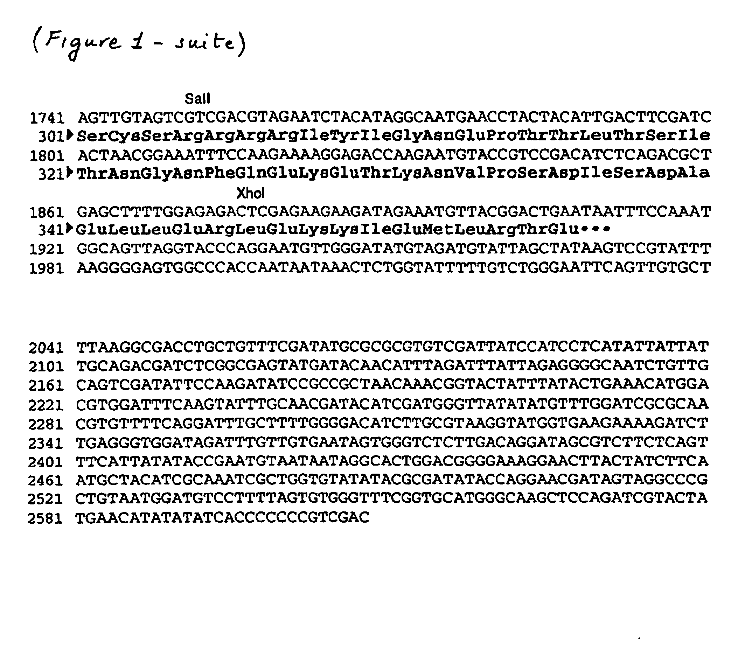

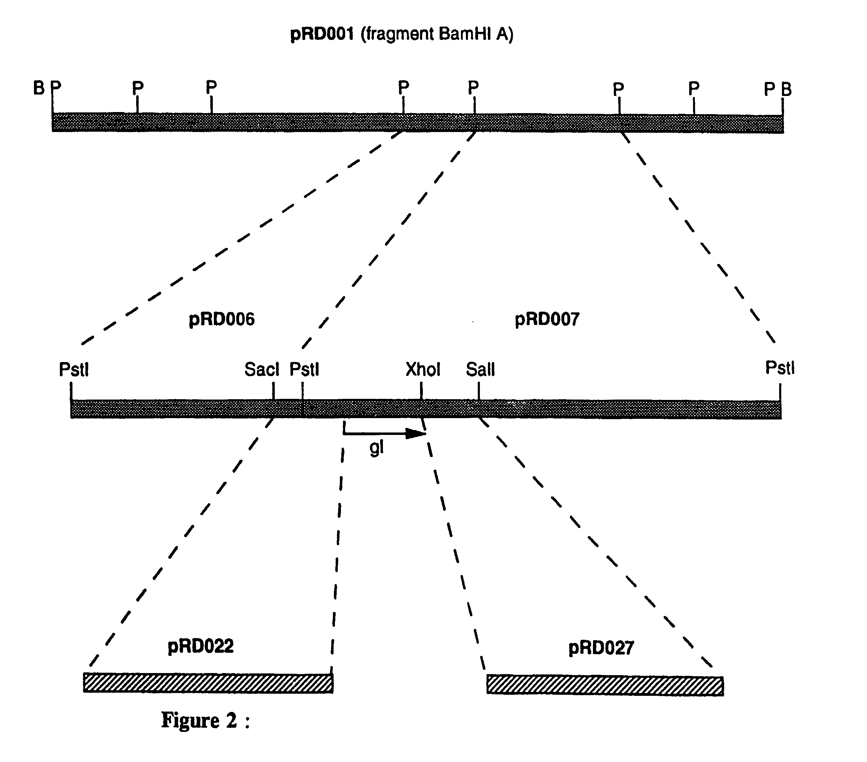

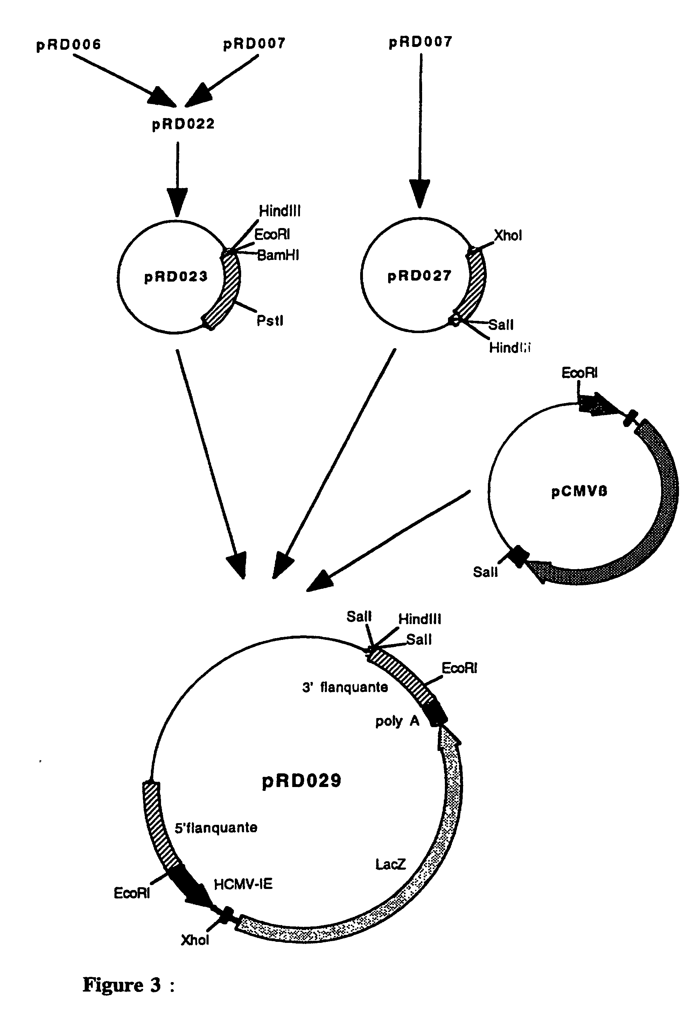

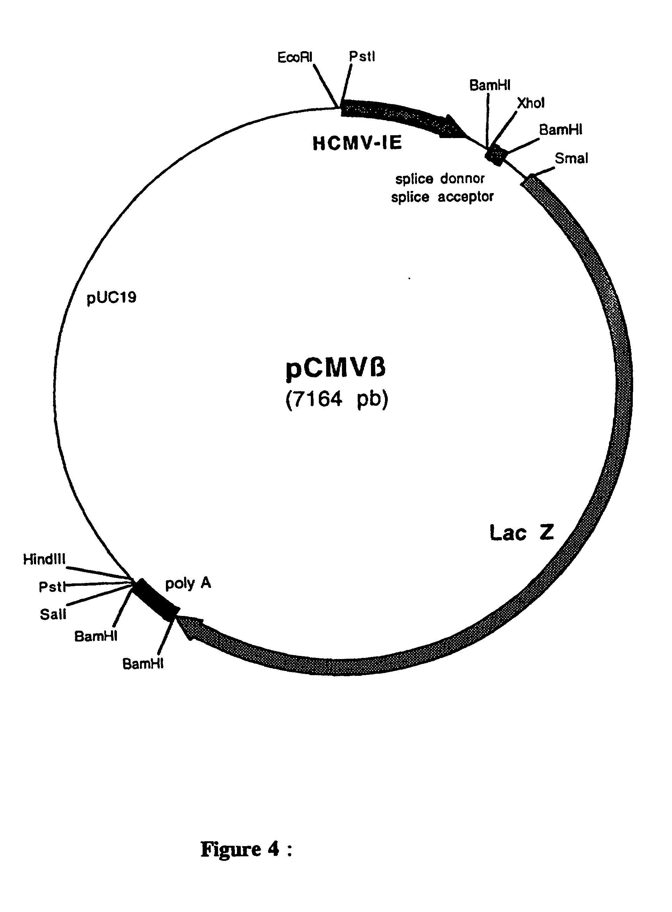

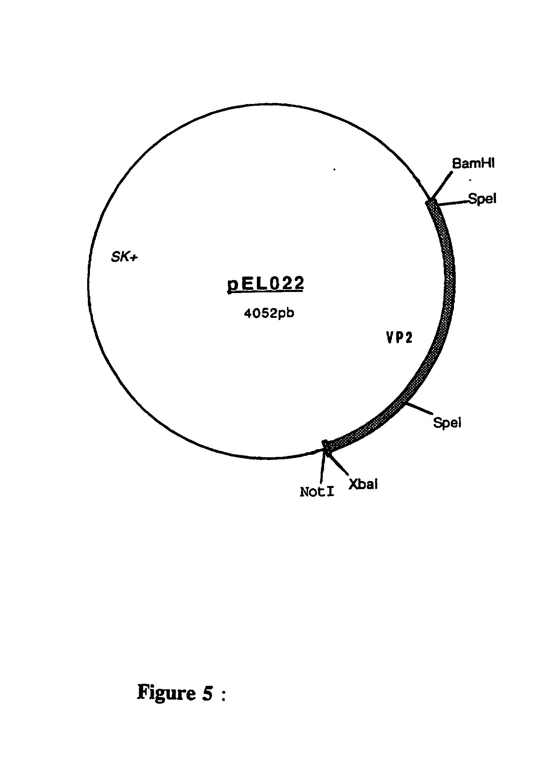

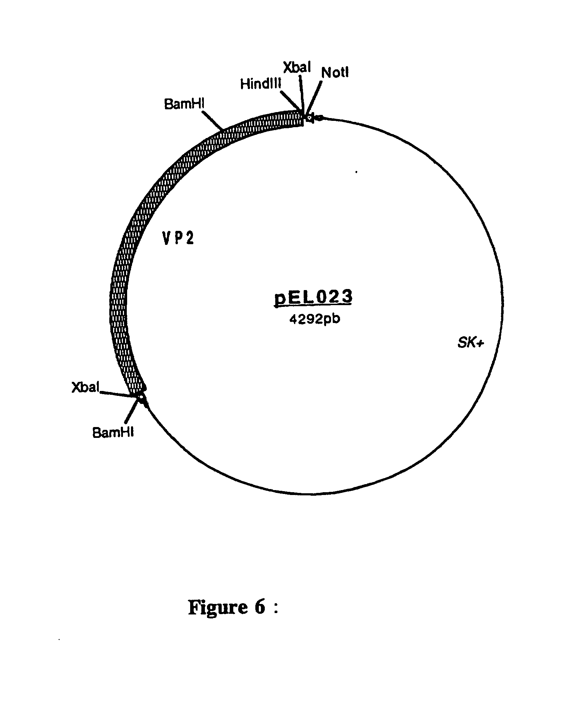

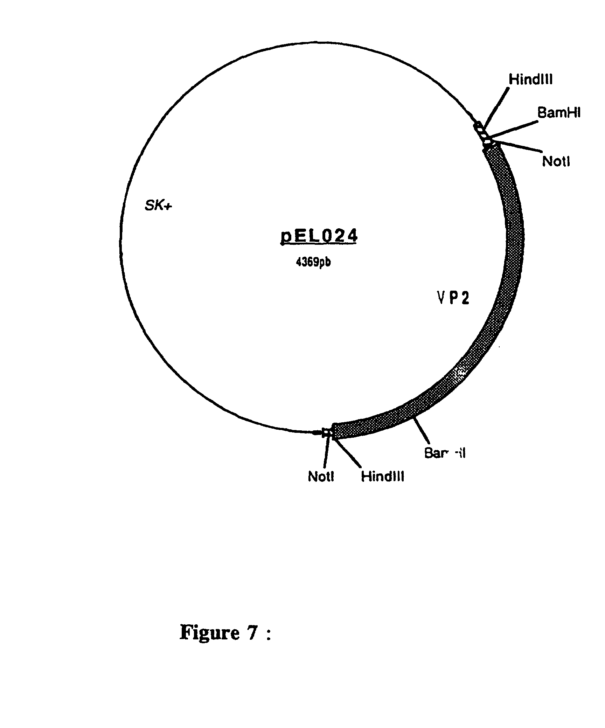

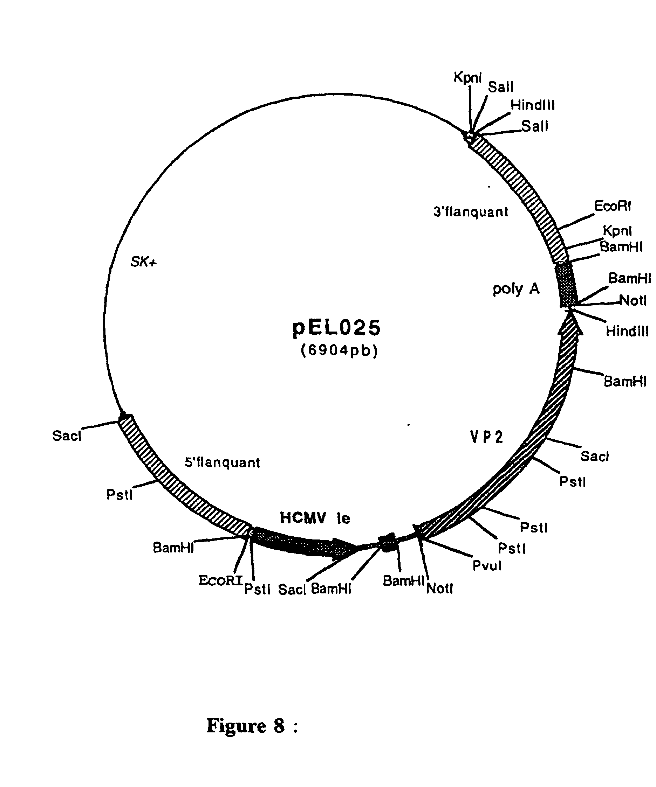

Le fragment BamHI A de 29 kpb du virus HVT souche FC126 (Igarashi T. *et al.* Virology. 1989. **70**. 1789-1804) a été cloné dans le site BamHI du vecteur pBR322 pour donner le plasmide pRD001. Le plasmide pRD001 a été digéré par Pstl et les fragments Pstl-Pstl 2,8 kpb et 5,7 kpb ont été clonés dans le site Pstl du vecteur pBR322 pour donner respectivement les plasmides pRD006 et pRD007. Le plasmide pRD006 a été digéré par Pstl et Sacl pour isoler le fragment Pstl-Sacl de 340 pb (fragment A).
Une PCR a été réalisée avec les oligonucléotides: 
RD045 (SEQ ID N° 1)
5′ TGCTGGTACCGTCGACAAGCTTGGATCCGTGCAGATAACACGTACTGGC
3′
pBRPst- (SEQ ID N° 2) 5′ CATGTAACTCGCCTTGATC 3′
 et la matrice pRD007 pour obtenir un fragment de 550 pb (positions (339) à 831 sur la figure 1 (SEQ ID N° 3) (positions 6491 à 6980 sur la séquence HVT U_{S} (Zelnik V. *et al.* J. Gen. Virol.1993. **74**. 2151-2162). Le fragment PCR de 520 pb a été ensuite digéré par Kpnl et Pstl pour isoler un fragment Pstl-Kpnl de 520 pb (fragment B). Les fragments A et B ont été ligaturés ensemble avec le vecteur pBS-SK + (Stratagene), préalablement digéré par Kpnl et Sacl pour donner le plasmide pRD022 (figure 2). Le plasmide pRD007 a été digéré par Sall et Xhol pour isoler le fragment Sall-Xhol de 730 pb (positions 1876 à 2608 sur SEQ ID N° 1) (positions 6491 à 6980 sur la séquence HVT U_{S} (Zelnik V. *et al.* J. Gen. Virol.1993. **74**. 2151-2162). Ce fragment a été cloné dans le site Sall du vecteur pBS-SK + pour donner le plasmide pRD027 (figure 2).
Un oligonucléotide synthétique double brin a été obtenu par hybridation des deux oligonucléotides suivants: 
RD048 (SEQ ID N° 4) 5′ GATCCAGCTGAATTCAGCTA 3′
RD049 (SEQ ID N° 5) 5′ AGCTTAAGCTGAATTCAGCTG 3′
 Cet oligonucléotide a été cloné entre les sites BamHI et Hindlll du plasmide pRD022 pour donner le plasmide pRD023 (figure 3). Le plasmide pCMVβ (Clontech Cat# 6177-1) (figure 4) a été digéré par EcoRI et Sall pour isoler le fragment EcoRI-SalI de 4500 pb contenant la cassette d'expression HCMV-IE = *IacZ* (fragment C). Le plasmide pRD027 a été digéré par Hindlll et Xhol pour isoler le fragment Hindlll-Xhol de 730 pb (fragment D). Les fragments C et D ont été ligaturés ensemble avec le plasmide pRD023, préalablement digéré par EcoRI et Hindlll, pour donner le plasmide pRD029 de 8973 pb (figure 3). Ce plasmide contient la cassette d'expression HCMV-IE = *IacZ* dans le site gl (délétion complète) du virus HVT.
Le plasmide pEL004 (= plasmide pGH004 décrit dans la demande de brevet français 92.13109) contenant le gène IBDV VP2 sous forme d'une cassette BamHI-HindIII a été digéré par BamHI et Xbal pour isoler le fragment BamHI-Xbal (gène VP2 tronqué) de 1104 pb. Ce fragment a été cloné dans le vecteur pBS-SK + (Stratagene), préalablement digéré avec Xbal et BamHI pour donner le plasmide pEL022 de 4052 pb (figure 5). Le vecteur pBS-SK + a été digéré par EcoRV et Xbal, puis ligaturé sur lui-même pour donner pBS-SK* (modifié). Le plasmide pEL004 a été digéré par Kpnl et Hindlll pour isoler le fragment Kpnl-Hindlll de 1387 pb contenant le gène IBDV VP2 complet. Ce fragment a été cloné dans le vecteur pBS-SK*, préalablement digéré par Kpnl et Hindlll, pour donner le plasmide pEL023 de 4292 pb (figure 6). Le plasmide pEL022 a été digéré par BamHI et Notl pour isoler le fragment BamHI-NotI de 1122 pb (fragment A). Le plasmide pEL023 a été digéré par BamHI et NotI pour isoler le fragment BamHI-NotI de 333 pb (fragment B). Les fragments A et B ont été ligaturés ensemble avec le vecteur pBS-SK +, préalablement digéré par Notl et traité avec la phosphatase alcaline, pour donner le plasmide pEL024 de 4369 pb (figure 7). Le plasmide pEL024 a été ensuite digéré par Notl pour isoler le fragment Notl-Notl de 1445 pb. Ce fragment a été cloné dans le plasmide pRD029, préalablement digéré par Notl et traité avec la phosphatase alcaline, pour donner le plasmide pEL025 de 6904 pb (figure 8).

### 5.2. Isolement et purification du recombinant vHVT2

Le plasmide pEL025 a été digéré par Sall pour linéarisation, puis extrait avec un mélange phénol/chloroforme (19:1), précipité avec de l'éthanol absolu, et repris dans de l'eau stérile.
Des cellules CEP primaires de 24 heures ont ensuite été transfectées avec le mélange suivant: 1 µg de plasmide pEL025 linéarisé + 5 µg d'ADN viral d'HVT dans 300 µl de milieu OptiMEM (Gibco BRL Cat# 041-01985H) et 100 µg de LipofectAMINE dilués dans 300 µl de milieu (volume final du mélange = 600 µl). Ces 600µl ont ensuite été dilués dans 3 ml (volume final) de milieu et étalés sur 3.10⁶ CEP I. Le mélange a été laissé en contact avec les cellules pendant 5 heures, puis éliminé et remplacé par 5 ml de milieu de culture. Les cellules ont alors été laissées en culture pendant 3 jours à + 37°C, puis elles ont été pronasées, mélangées à des CEP II fraîches (mélange 3:1), et réétalées sur 1 plaque 96 puits. Cette plaque a été laissée en culture pendant 3 jours, puis les cellules ont été pronasées, mélangées à des CEP II fraîches, et réétalées sur 2 plaques de 96 puits, une cupule initiale donnant 2 cupules soeurs. Les plaques 96 puits ont été mises en culture jusqu'à apparition d'un effet cytopathique. Après 72 heures de culture, une des deux plaques 96 puits a été fixée à l'acétone 95% pendant 30 minutes et une réaction d'immunofluorescence indirecte (IFI) a été réalisée avec un anticorps monoclonal anti-VP2 pour rechercher les plages exprimant la protéine VP2. Les cupules "soeurs" des cupules présentant des plages positives en IFI ont été pronasées, mélangées à des CEP II fraîches, et déposées en dilution limite sur des plaques 96 puits. Après 3 jours de culture, les cupules présentant un effet cytopathique ont été pronasées, mélangées à des CEP II, et réétalées sur des plaques 96 puits, une cupule initiale donnant 2 cupules soeurs. 3 jours après, les plages exprimant la protéine VP2 ont été à nouveau recherchées comme précédemment par IFI sur l'une des 2 plaques soeurs.
En général, 4 cycles d'isolement successifs (récolte d'une cupule, réétalement, contrôle par IFI, repiquage d'un cupule soeur...) suffisent pour obtenir des virus recombinants dont la totalité de la progénie présente une fluorescence spécifique. Une plage virale ayant donné 100 % de plages positives en IFI avec un anticorps monoclonal anti-VP2 a été désignée vHVT2. L'ADN génomique de ce virus recombinant a été caractérisé au niveau moléculaire par des techniques classiques de PCR et de Southern blot en utilisant les oligonucléotides et les sondes d'ADN appropriés. Ce recombinant contient une cassette HCMV-IE/IBDV VP2 à la place du gène gl du virus HVT.

### Exemple 6: Construction du plasmide donneur pEL042 et isolement de vHVT4

### 6.1. Construction du plasmide donneur pEL042

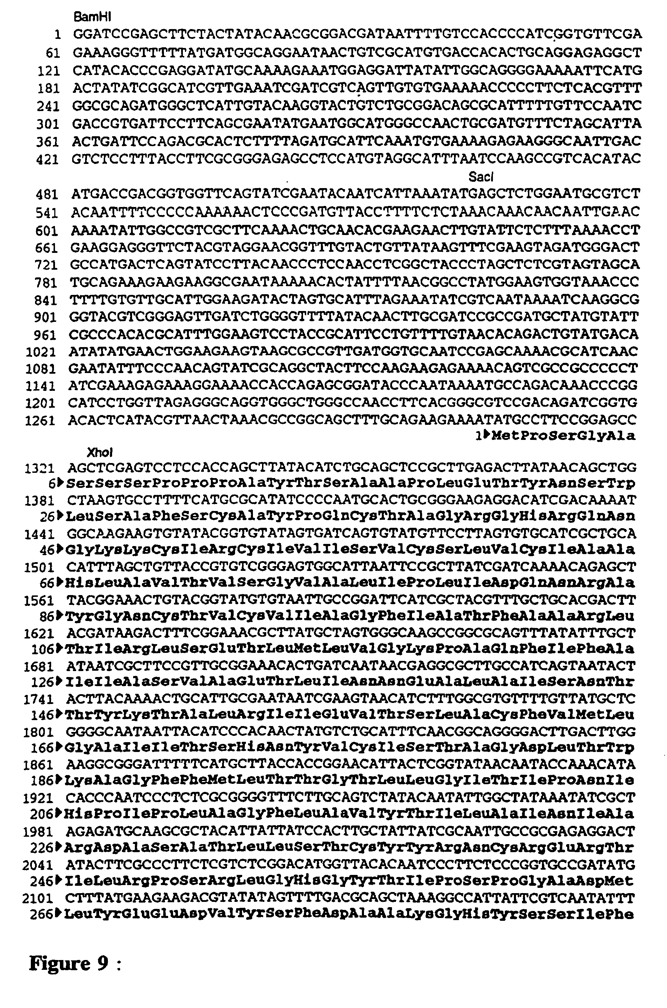

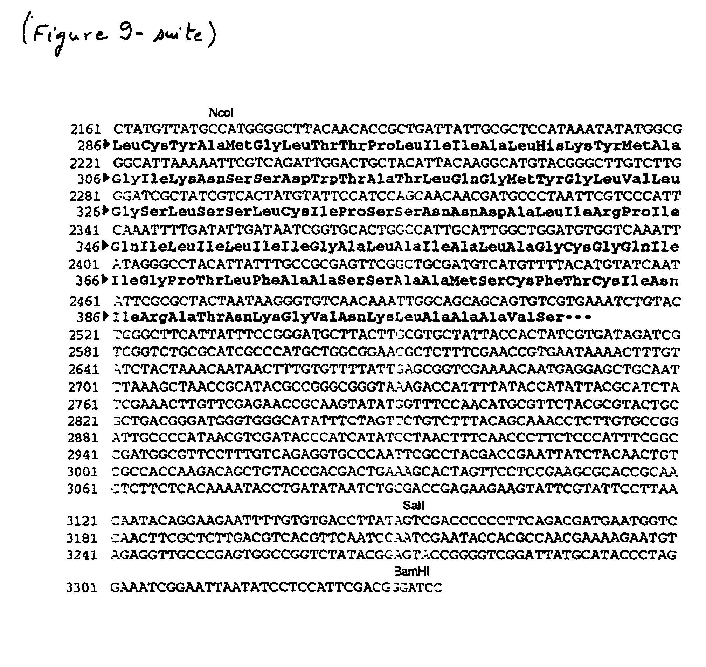

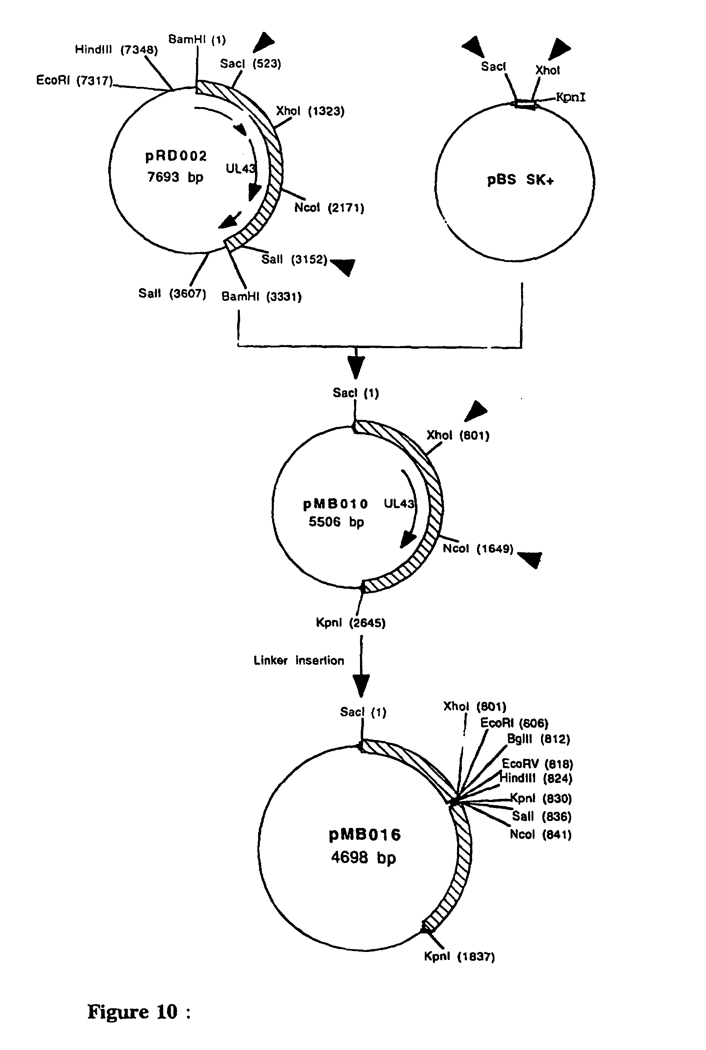

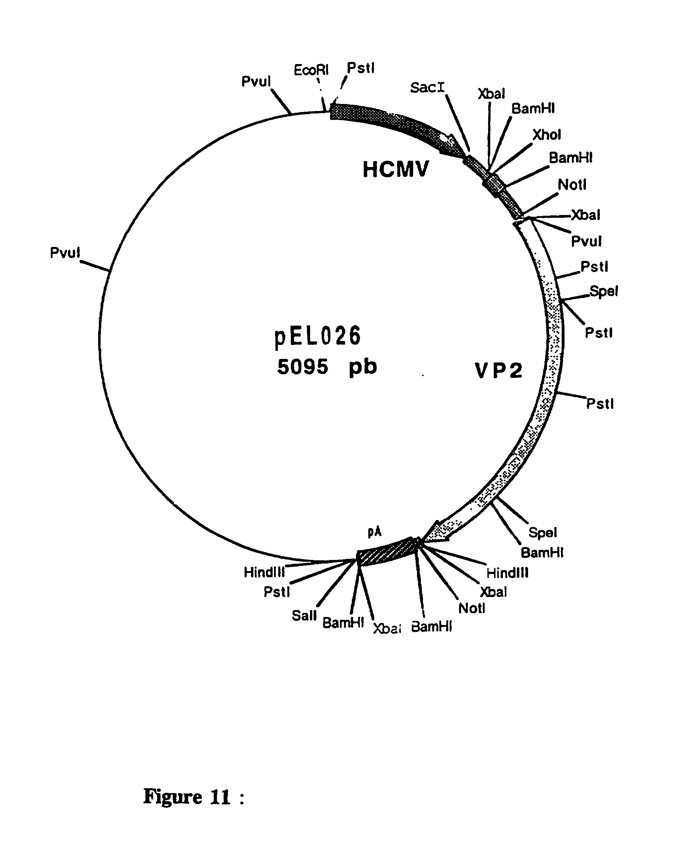

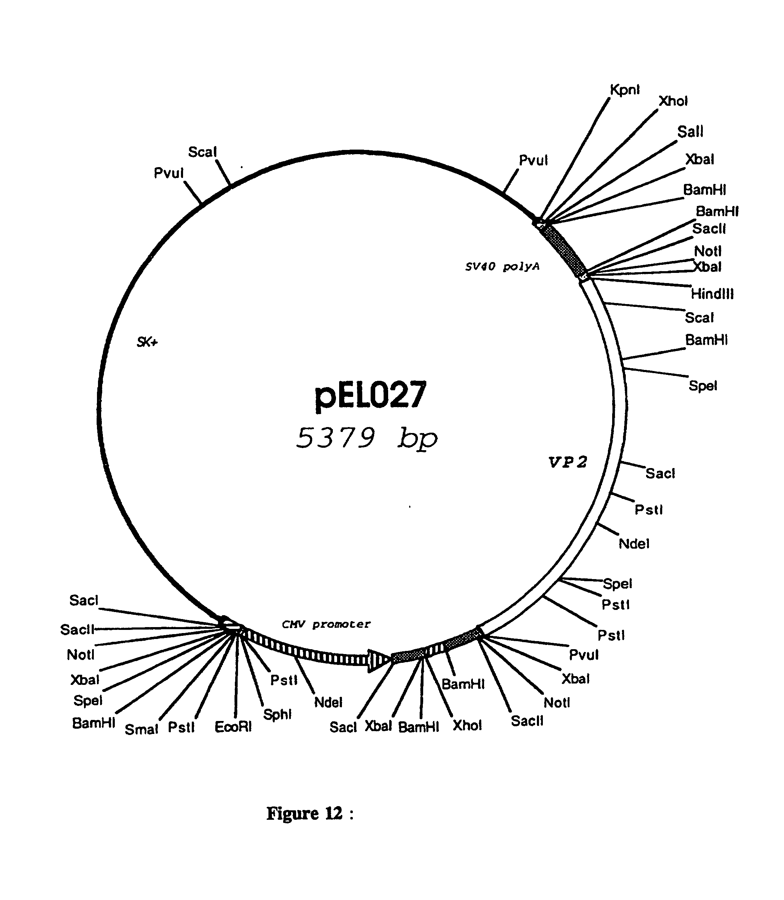

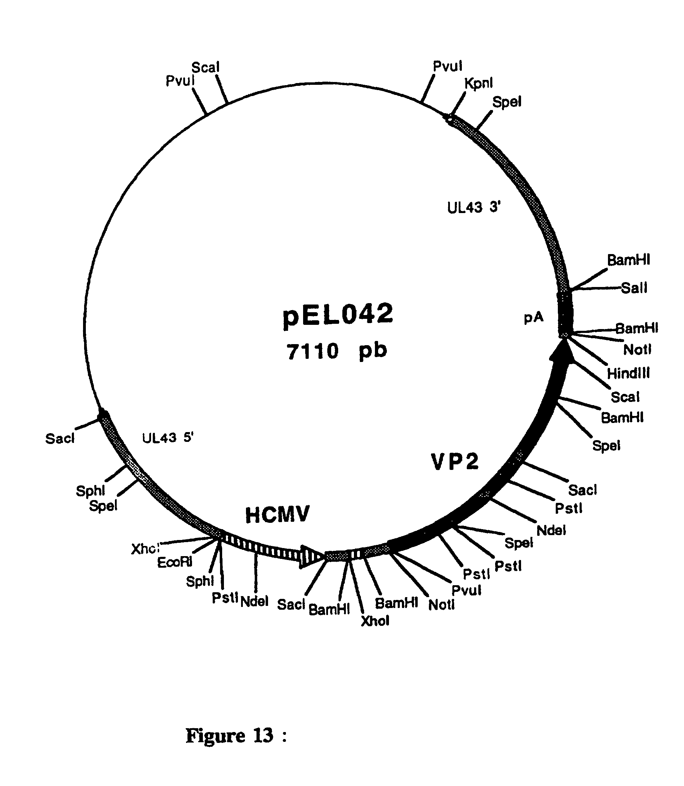

Le fragment BamHI M de 3,3 kpb du génome du virus HVT souche FC126 (Igarashi T. *et al.* Virology. 1989. **70**. 1789-1804) a été cloné dans le site BamHI du vecteur pBR322 pour donner le plasmide pRD002. La séquence du fragment BamHI M a été entièrement établie (3336 pb) (SEQ ID N° 6 et figure 9). Cette séquence contient une ORF codant pour une protéine homologue du produit du gène HSV-1 UL43 (position 1306 à 2511 (fin du codon stop) sur la séquence SEQ ID N° 6, figure 9). La protéine théoriquement codée par cette ORF a une taille de 401 acides aminés (aa). Le plasmide pRD002 a été digéré par Sacl et Sall pour isoler le fragment Sacl-Sall de 2620 pb. Ce fragment a été ligaturé dans le vecteur pBS-SK +, préalablement digéré par Sacl et Xhol, pour donner le plasmide pMB01 0 de 5506 pb (figure 10). Le plasmide pMB01 O a été ensuite digéré par Ncol et Xhol pour isoler le fragment Ncol-Xhol de 4650 pb. Ce fragment a été ligaturé avec un oligonucléotide synthétique double brin obtenu par hybridation des 2 oligonucléotides suivants: 
MB014 (SEQ ID N° 7)
5' TCGAGAATTCAGATCTGATATCAAGCTTGGTACCGTCGAC 3'
 
MB015 (SEQ ID N° 8)
5' CATGGTCGACGGTACCAAGCTTGATATCAGATCTGAATTC 3'
 pour donner le plasmide pMB016 de 4698 pb (figure 10). L'oligonucléotide inséré contient des sites de restriction permettant l'insertion d'une cassette d'expression entre les deux bras flanquants pour le site UL43. Le bras flanquant 5' a une taille de 800 pb (position 523 à position 1323 sur la séquence du fragment BamHI M (SEQ ID N° 6). Le bras 3' a une taille de 981 pb (position 2171 à 3152 sur SEQ ID N° 6). La délétion ainsi obtenue dans le gène HVT UL43 va de la position 1324 à la position 2170 (délétion de 847 nucléotides, soit 282 aa sur 401 aa au total). Le plasmide pEL024 (voir exemple 5) a été digéré par Notl pour isoler le fragment Notl-Notl de 1445 pb. Ce fragment a été ligaturé avec le plasmide pCMVβ, préalablement digéré par Notl, pour donner le plasmide pEL026 de 5095 pb (figure 11). Le plasmide pEL026 a été digéré par EcoRI, Sall et Xmnl pour isoler le fragment EcoRI-Sall de 2428 pb. Ce fragment a été ligaturé avec le vecteur pBS-SK +, préalablement digéré par EcoRI et Sall, pour donner le plasmide pEL027 de 5379 pb (figure 12). Le plasmide pEL027 a été digéré par EcoRI, Sall et Xmnl pour isoler le fragment EcoRI-Sall de 2428 pb. Ce fragment a été ligaturé dans le plasmide pMB016, préalablement digéré par EcoRI et Sall, pour donner le plasmide pEL042 de 7110 pb (figure 13).

### 6.2. Isolement et purification du recombinant vHVT4

Une co-transfection de CEP Il avec le plasmide pEL042 linéarisé par Kpnl et l'ADN viral d'HVT a été réalisée comme décrit dans l'exemple 5. Les conditions de tranfection, d'isolement et de purification des plages virale recombinantes issues de cette co-transfection ont été celles décrites dans l'exemple 5.
Une plage virale ayant donné 100 % de plages positives en IFI avec un anticorps monoclonal anti-IBDV VP2 a été désignée vHVT4. L'ADN génomique de ce virus recombinant a été caractérisé au niveau moléculaire par des techniques classiques de PCR et de Southern blot en utilisant les oligonucléotides et les sondes d'ADN appropriés.

### Exemple 7: Construction du plasmide donneur pEL072 et isolement de vHVT6

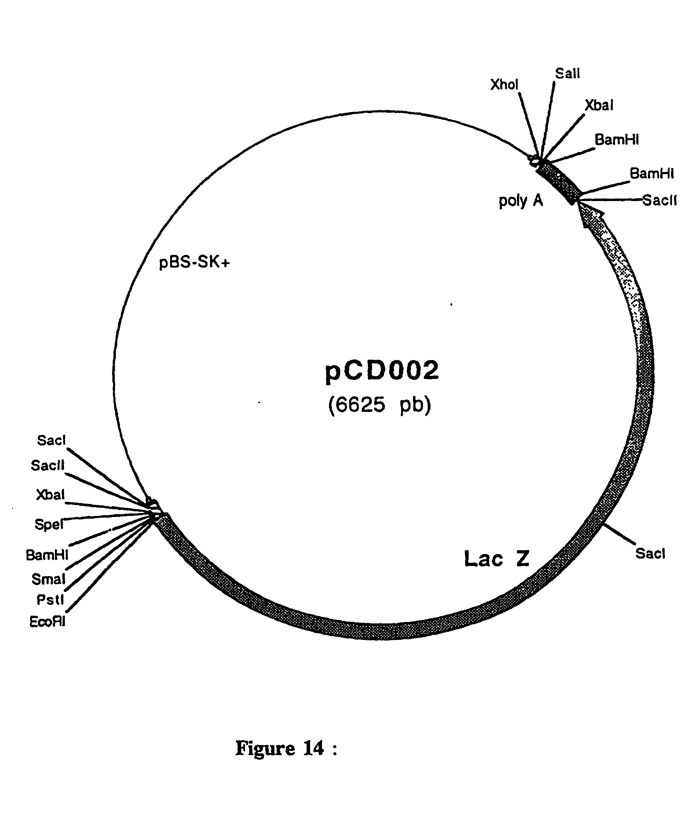

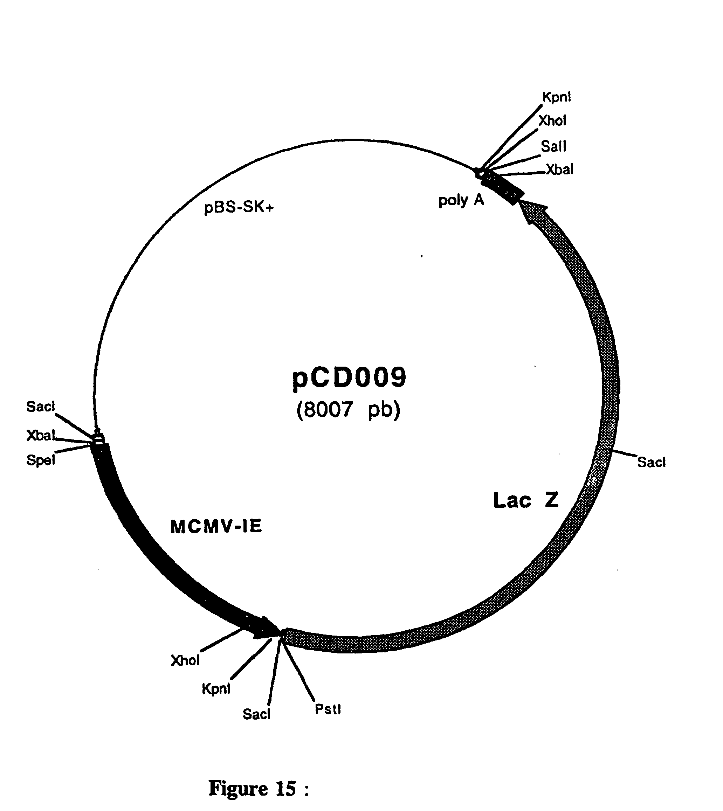

Le plasmide pCMVβ (figure 4) a été digéré par Sall et Smal pour isoler le fragment SalI-SmaI de 3679 pb contenant le gène *lacZ* ainsi que le signal de poly-adénylation du gène tardif du virus SV40. Ce fragment a été inséré dans le vecteur pBS-SK +, préalablement digéré par Sall et EcoRV, pour donner le plasmide pCD002 de 6625 pb (figure 14). Ce plasmide contient le gène reporter *lacZ* mais aucun promoteur n'est situé en amont de ce gène. L'ADN génomique viral du virus MCMV a été préparé comme décrit dans l'exemple 2, et digéré par PstI pour isoler le fragment Pstl-Pstl de 2285 pb. Ce fragment a été cloné dans le vecteur pBS-SK +, préalablement digéré par Pstl et traité avec la phosphatase alcaline, pour donner le plasmide pCD004. Le plasmide pCD004 a été digéré par Hpal et Pstl pour isoler le fragment Hpal-Pstl de 1389 pb qui contient la région promotrice/activatrice du gène Immediate-Early du cytomégalovirus murin (Murine CytoMegaloVirus = MCMV) (Dorseh-Häsler K. *et al.* Proc. Natl. Acad. Sci. 1985. **82**. 8325-8329, et demande de brevet WO-A-87/03905). Ce fragment a été cloné dans le plasmide pCD002, préalablement digéré par Pstl et Smal, pour donner le plasmide pCD009 de 8007 pb (figure 15).
Un oligonucléotide double brin a été obtenu par hybridation des deux oligonucléotides suivants :

MB070 (SEQ ID N° 9)
5′ CGAATTCACTAGTGTGTGTCTGCAGGCGGCCGCGTGTGTGTCGACGGTAC 3′

MB071 (SEQ ID N° 10)
5' CGTCGACACACACGCGGCCGCCTGCAGACACACACTAGTGAATTCGAGCT 3'

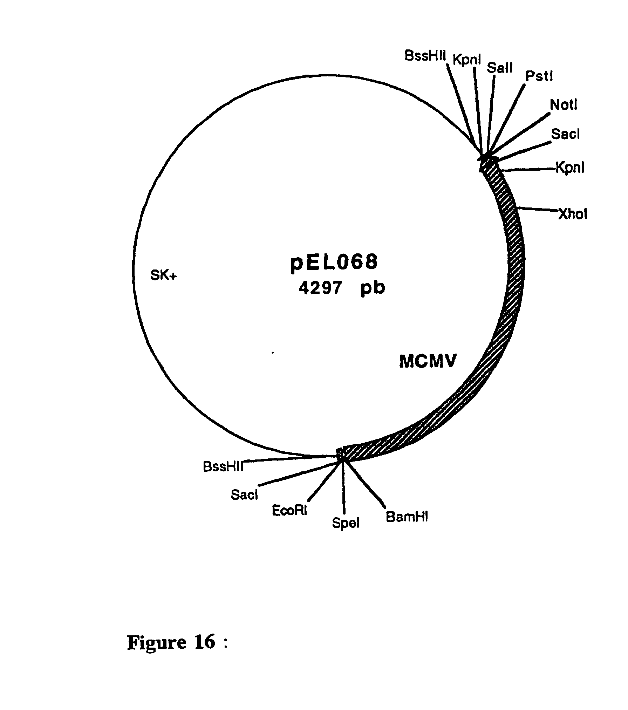

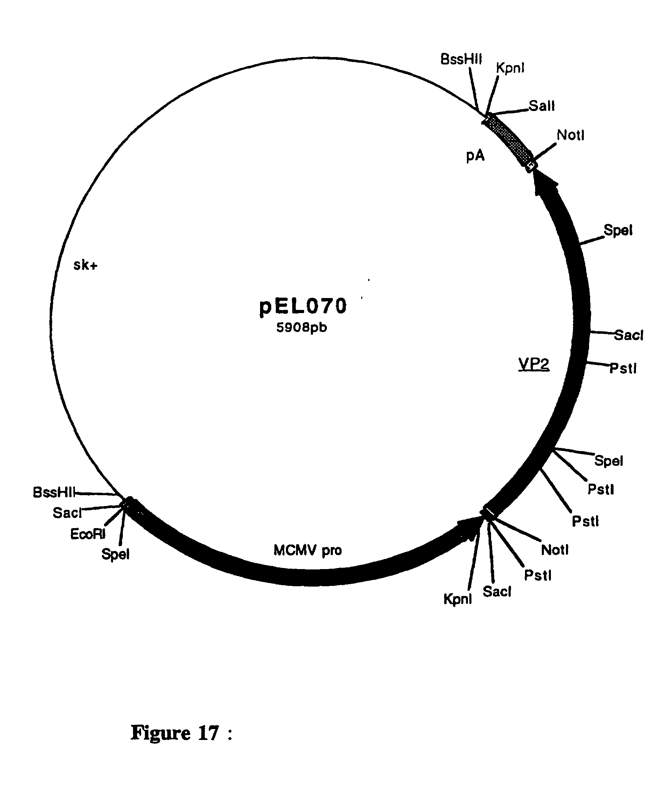

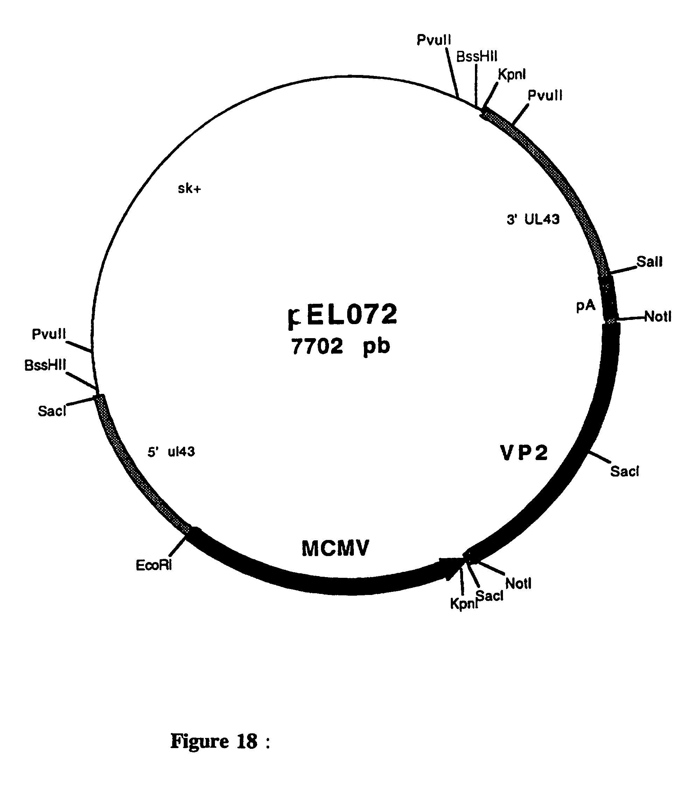

Cet oligonucléotide double brin a été ligaturé avec le vecteur pBS-SK +, préalablement digéré par Kpnl et Sacl, pour donner le plasmide pEL067. Le plasmide pCD009 a été digéré par Pstl et Spel pour isoler le fragment Pstl-Spel de 1396 pb. Ce fragment a été ligaturé avec le plasmide pEL067, préalablement digéré par Pstl et Spel, pour donner le plasmide pEL068 de 4297 pb (figure 16). Le plasmide pEL024 (voir exemple 5) a été digéré par Hindlll et Notl pour isoler le fragment Hindlll-Notl de 1390 pb (fragment A). Le plasmide pEL027 (voir exemple 6) a été digéré par Hindlll et Sall pour isoler le fragment Hindlll-Sall de 235 pb (fragment B). Les fragments A et B ont été ligaturés ensemble avec le plasmide pEL068, préalablement digéré par Notl et Sall, pour donner le plasmide pEL070 de 5908 pb (figure 17). Le plasmide pEL070 a été digéré par EcoRI, Sall et Xmnl pour isoler le fragment EcoRI-Sall de 3035 pb. Ce fragment a été ligaturé avec la plasmide pMB016 (voir exemple 6), préalablement digéré par EcoRI et Sall, pour donner le plasmide pEL072 de 7702 pb (figure 18). Ce plasmide permet l'insertion de la cassette d'expression MCMV-IE/IBDV VP2 dans le site UL43 du virus HVT.
Une co-transfection réalisée, comme décrit dans l'exemple 5, avec le plasmide pEL072 et l'ADN génomique du virus HVT a conduit à l'isolement et à la purification du recombinant vHVT6.

### Exemple 8: Construction du plasmide donneur pCD012 et isolement de vHVT7

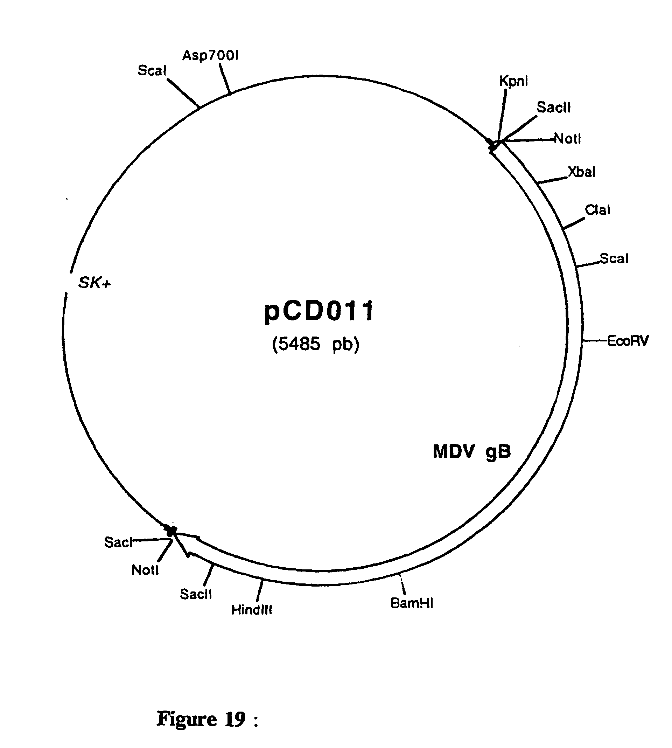

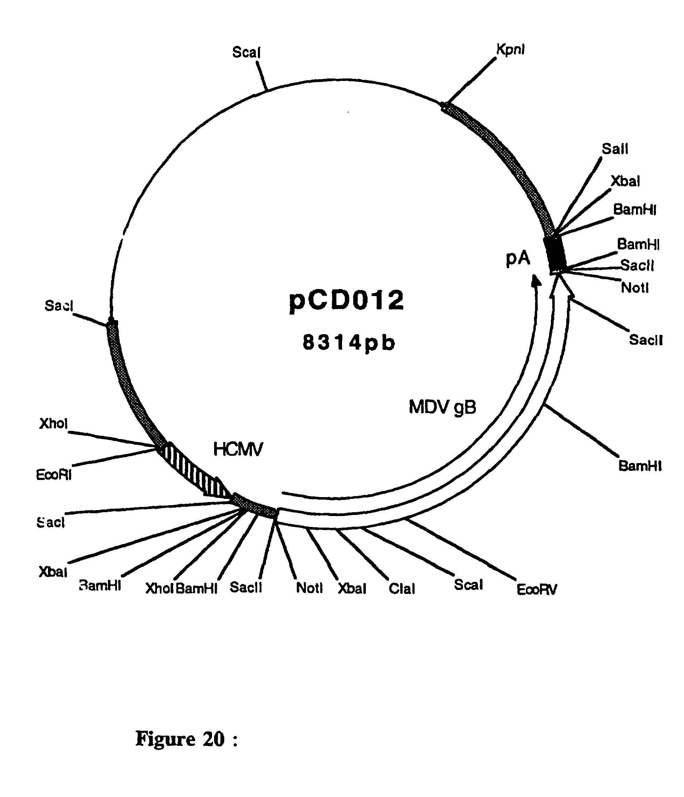

Le fragment EcoRI-SalI de 3,9 kpb de l'ADN génomique du virus MDV souche RB1B contenant le gène MDV gB (séquence publiée par Ross N. *et al.* J. Gen. Virol. 1989. **70**. 1789-1804) a été ligaturé avec le vecteur pUC13, préalablement digéré par EcoRI et SalI, pour donner le plasmide pCD007. Ce plasmide a été digéré par Sacl et Xhol pour isoler le fragment Sacl-Xhol de 2260 pb (partie centrale du gène gB = fragment A). Une PCR a été réalisée avec les oligonucléotides suivants: 
CD001 (SEQ ID N° 11)
5' GACTGGTACCGCGGCCGCATGCACTTTTTAGGCGGAATTG 3'
 
[?]

CD002 (SEQ ID N° 12) 5' TTCGGGACATTTTCGCGG 3'
 et la matrice pCD007 pour produire un fragment PCR de 222 pb. Ce fragment a été digéré par Kpnl et Xbal pour isoler un fragment Kpnl-Xbal de 190 pb (extrémité 5' du gène gB = fragment B). Une autre PCR a été réalisée avec les oligonucléotides suivants: CD003 (SEQ ID N° 13) 5' TATATGGCGTTAGTCTCC 3' 
CD004 (SEQ ID N° 14)
5' TTGCGAGCTCGCGGCCGCTTATTACACAGCATCATCTTCTG 3'
 et la matrice pCD007 pour produire un fragment PCR de 195 pb. Ce fragment a été digéré par SacI et SacII pour isoler le fragment Sacl-Sacll de 162 pb (extrémité 3' du gène gB = fragment C). Les fragments A, B et C ont été ligaturés ensemble avec le vecteur pBS-SK +, préalablement digéré par Kpnl et Sacl, pour donner le plasmide pCD011 de 5485 pb (figure 19). Le plasmide pCD011 a été digéré par Notl pour isoler le fragment Notl-Notl de 2608 pb (gène MDV gB entier = fragment D). Le plasmide pEL042 (voir exemple 6) a été digéré par Notl et traité avec la phosphatase alcaline pour isoler le fragment Notl-Notl de 5706 pb (fragment E). Les fragments D et E ont alors été ligaturés ensemble pour donner le plasmide pCD012 de 8314 pb (figure 20). Ce plasmide permet l'insertion de la cassette HCMV-IE/MDV gB dans le site UL43 du virus HVT.
Une co-transfection réalisée, comme décrit dans l'exemple 5, avec le plasmide pCD012 et l'ADN génomique du virus HVT a conduit à l'isolement et à la purification du recombinant vHVT7.

### Exemple 9: Construction du plasmide donneur pEL043 et isolement de vHVT8

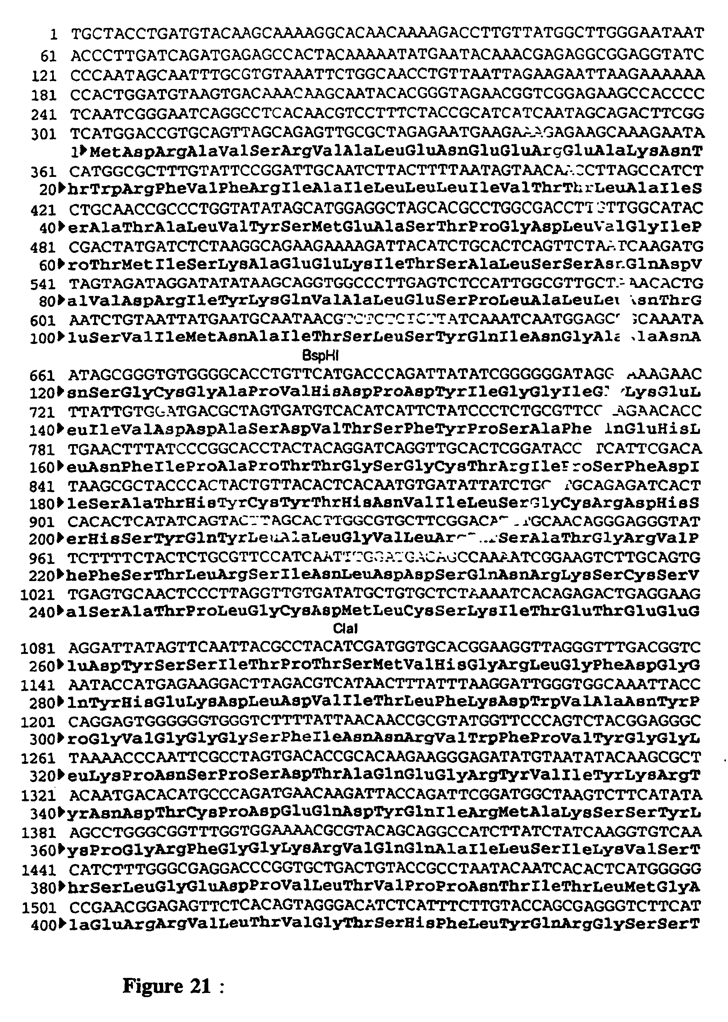

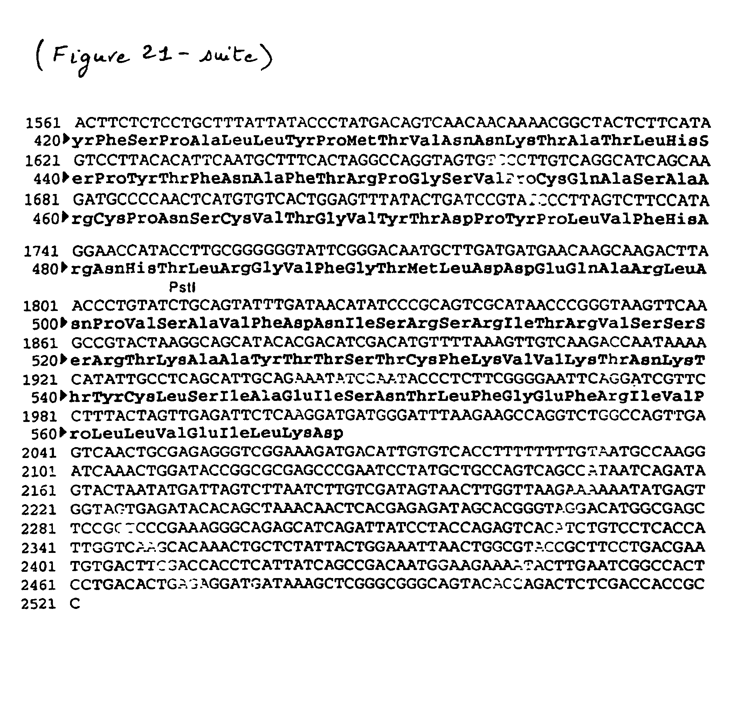

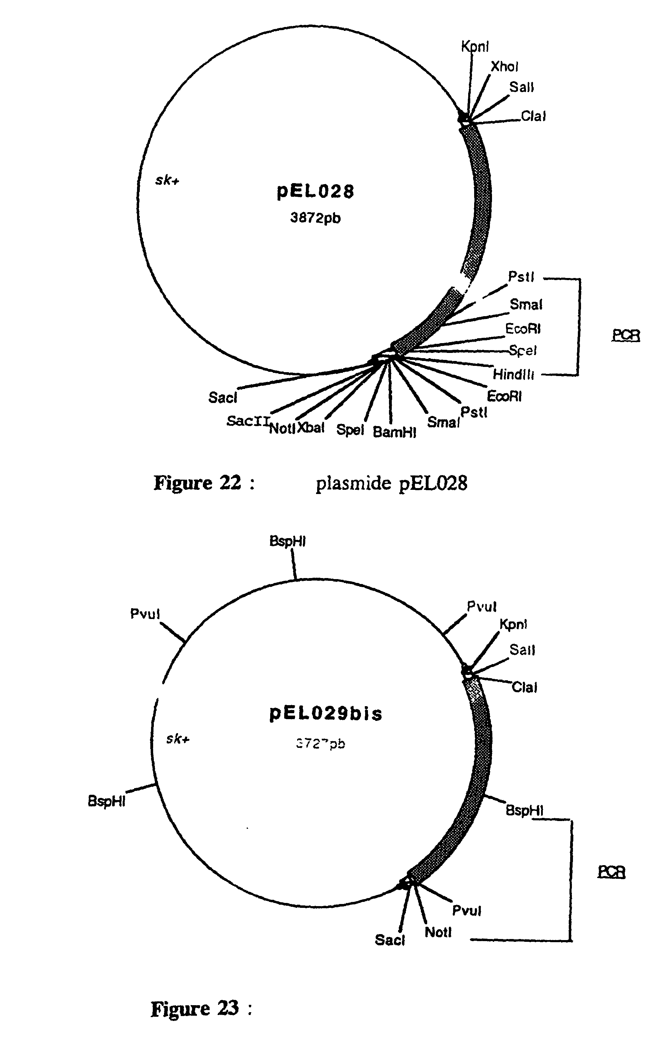

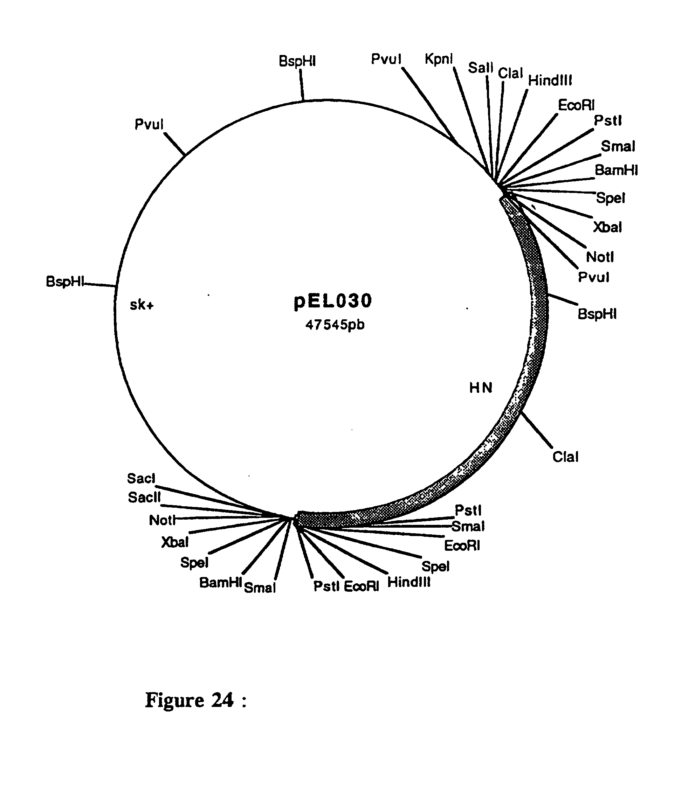

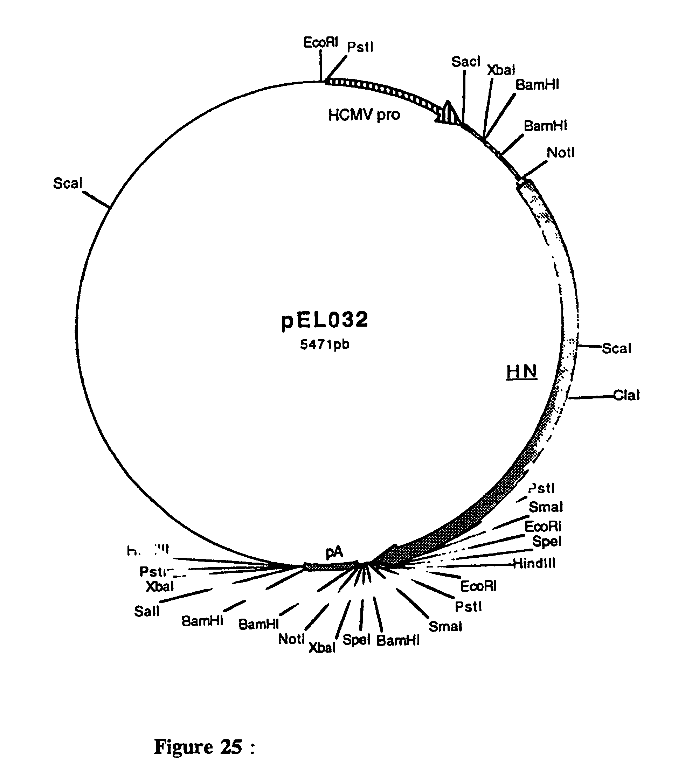

La constitution d'une banque d'ADN complémentaire du génome du virus de la maladie de Newcastle (NDV), souche Texas, a été réalisée comme décrit par Taylor J. *et al*. (J. Virol. 1990. **64**. 1441-1450). Un clone pBR322 contenant la fin du gène fusion (F), la totalité du gène hémagglutinine-neuraminidase (HN) et le début du gène de la polymérase a été identifié pHN01. La séquence du gène NDV HN contenue sur ce clone est présentée sur la figure 21 (SEQ ID N° 15). Le plasmide pHN01 a été digéré par Sphl et Xbal pour isoler le fragment Sphl-Xbal de 2520 pb. Ce fragment a été ligaturé avec le vecteur pUC19, préalablement digéré par Sphl et Xbal, pour donner le plasmide pHN02 de 5192 pb. Le plasmide pHN02 a été digéré par ClaI et Pstl pour isoler le fragment Clal-Pstl de 700 pb (fragment A). Une PCR a été réalisée avec les oligonucléotides suivants: EL071 (SEQ ID N° 16) 5′ CAGACCAAGCTTCTTAAATCCC 3′ EL073 (SEQ ID N° 17) 5′ GTATTCGGGACAATGC 3′ et la matrice pHN02 pour produire un fragment PCR de 270 pb. Ce fragment a été digéré par Hindlll et Pstl pour isoler un fragment Hindlll-Pstl de 220 pb (fragment B). Les fragments A et B ont été ligaturés ensemble avec le vecteur pBS-SK +, préalablement digéré par Clal et Hindlll, pour donner le plasmide pEL028 de 3872 pb (figure 22). Le plasmide pHN02 a été digéré par Bsphl et Clal pour isoler le fragment Bsphl-Clal de 425 pb (fragment C). Une PCR a été réalisée avec les oligonucléotides suivants: EL074 (SEQ ID N° 18) 5′ GTGACATCACTAGCGTCATCC 3′ 
EL075 (SEQ ID N° 19)
5' CCGCATCATCAGCGGCCGCGATCGGTCATGGACAGT 3'
 et la matrice pHN02 pour produire un fragment PCR de 465 pb. Ce fragment a été digéré par Bsphl et Notl pour isoler le fragment Bsphl-Notl de 390 pb (fragment D). Les fragments C et D ont été ligaturés ensemble avec le vecteur pBS-SK +, préalablement digéré par Clal et Notl, pour donner le plasmide pEL029bis de 3727 pb (figure 23). Le plasmide pEL028 a été digéré par Clal et Sacll pour isoler le fragment Clal-Sacll de 960 pb (fragment E). Le plasmide pEL029bis a été digéré par Clal et Notl pour isoler le fragment Clal-Notl de 820 pb (fragment F). Les fragments E et F ont été ligaturés ensemble avec le vecteur pBS-SK+, préalablement digéré par Notl et Sacll, pour donner le plasmide pEL030 de 4745 pb (figure 24). Le plasmide pEL030 a été digéré par Notl pour isoler le fragment Notl-Notl de 1780 pb (gène NDV HN entier). Ce fragment a été ligaturé, à la place du gène lacZ, avec le plasmide pCMVβ, préalablement digéré par Notl et traité avec la phophatase alcaline, pour donner le plasmide pEL032 de 5471 pb (figure 25). Le plasmide pEL032 a été digéré par EcoRI et Clal pour isoler le fragment EcoRI-Clal de 1636 pb (fragment G).

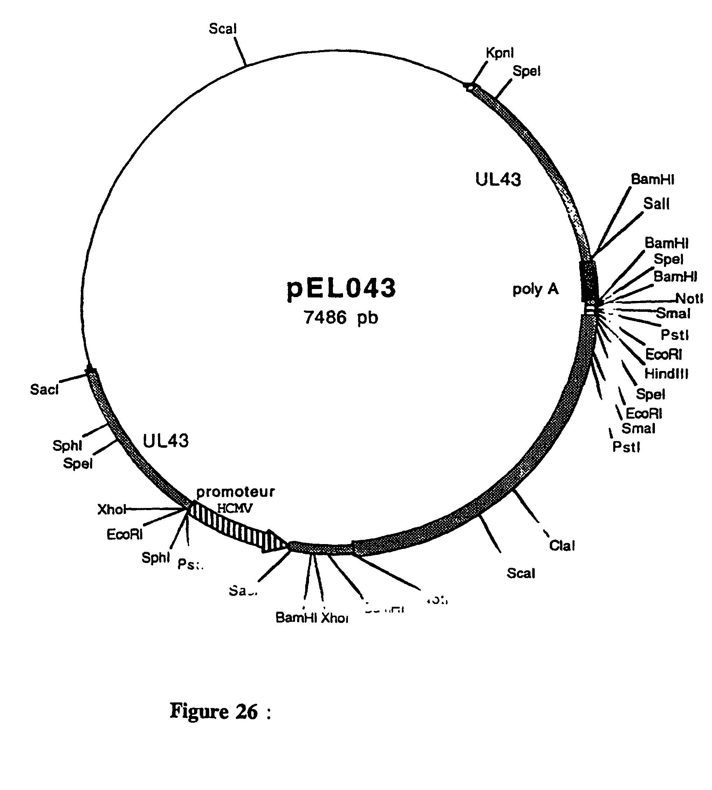

Le plasmide pEL032 a été digéré par Clal et Sall pour isoler le fragment Clal-Sall de 1182 pb (fragment H). Les fragments G et H ont été ligaturés ensemble avec le plasmide pMB016 (voir exemple 6), préalablement digéré par EcoRI et Sall, pour donner le plasmide pEL043 de 7486 pb (figure 26). Ce plasmide permet l'insertion de la cassette d'expression HCMV-IE/NDV HN dans le site UL43 du virus HVT.
Une co-transfection réalisée, comme décrit dans l'exemple 5, avec le plasmide pEL043 et l'ADN génomique du virus HVT a conduit à l'isolement et à la purification du recombinant vHVT8.

### Exemple 10: Construction du plasmide donneur pEL044 et isolement de vHVT9

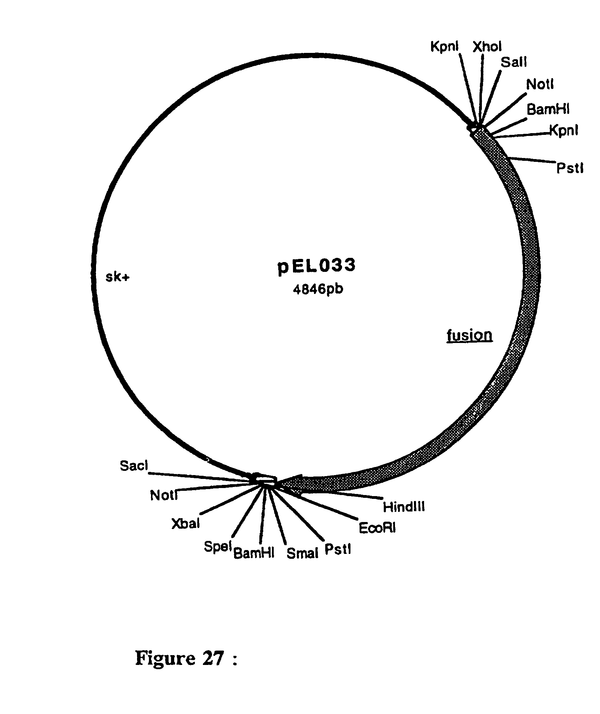

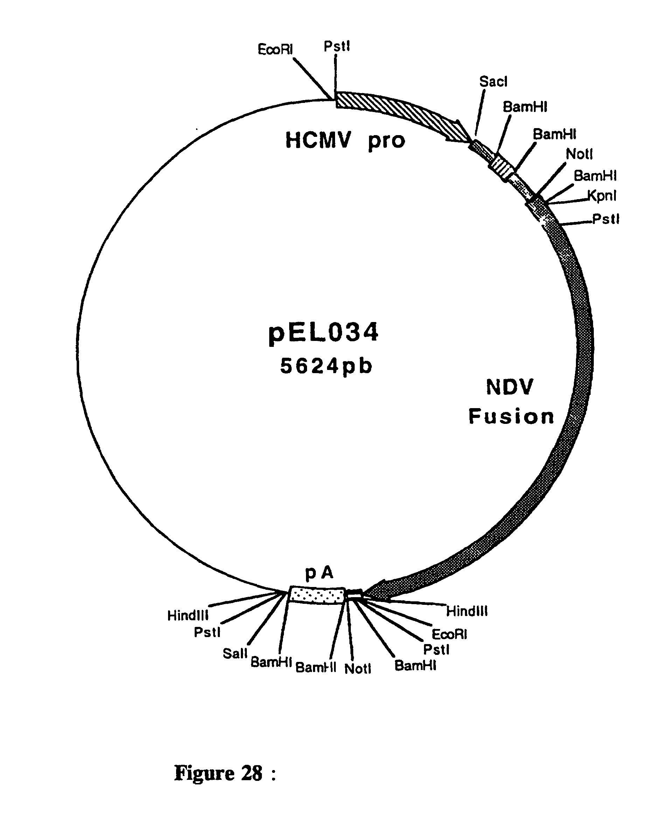

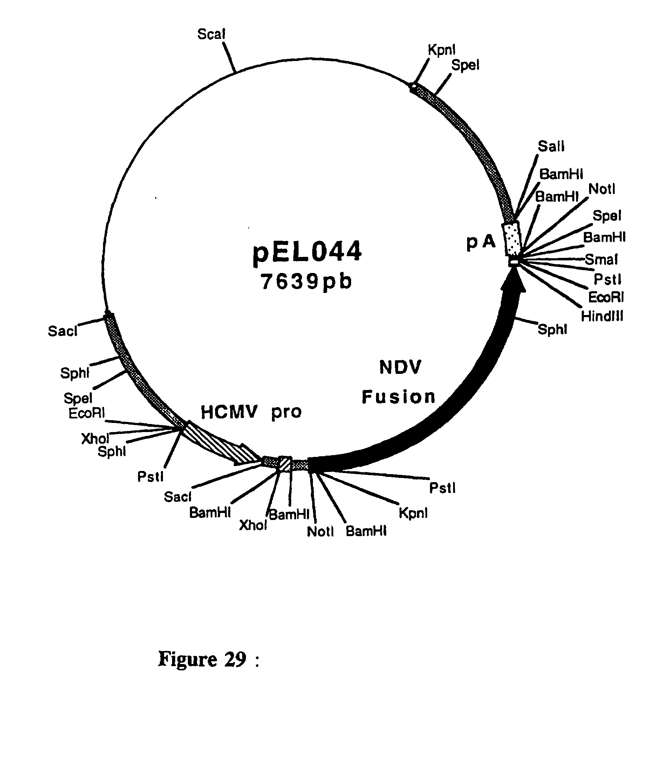

Un clone provenant de la banque d'ADN complémentaire du génome du virus de la maladie de Newcastle (voir exemple 9) et contenant le gène fusion (F) en entier a été appelé pNDV81. Ce plasmide a été décrit précédemment et la séquence du gène NDV F présent sur ce clone a été publiée (Taylor J. *et al.* J. Virol. 1990. **64**. 1441-1450). Le plasmide pNDV81 a été digéré par Narl et Pstl pour isoler le fragment Narl-Pstl de 1870 pb (fragment A). Une PCR a été réalisée avec les oligonucléotides suivants: EL076 (SEQ ID N° 20) 5' TGACCCTGTCTGGGATGA 3' 
EL077 (SEQ ID N° 21)
5' GGATCCCGGTCGACACATTGCGGCCGCAAGATGGGC 3'
 et la matrice pNDV81 pour produire un fragment de 160 pb. Ce fragment a été digéré par Pstl et Sall pour isoler le fragment Pstl-Sall de 130 pb (fragment B). Les fragments A et B ont été ligaturés ensemble avec le vecteur pBS-SK +, préalablement digéré par Clal et Sall, pour donner le plasmide pEL033 de 4846 pb (figure 27). Le plasmide pEL033 a été digéré par Notl pour isoler le fragment Notl-Notl de 1935 pb (gène F entier). Ce fragment a été ligaturé avec le plasmide pCMVβ, préalablement digéré par Notl et traité avec la phosphatase alcaline, pour donner le plasmide pEL034 de 5624 pb (le gène NDV F a remplacé le gène *lacZ)* (figure 28). Le plasmide pEL034 a été digéré par EcoRI et Kpnl pour isoler le fragment EcoRI-KpnI de 866 pb (fragment C). Le plasmide pEL034 a été digéré par Kpnl
et Sall pour isoler le fragment Kpnl-Sall de 2114 pb (fragment D). Les fragments C et D ont été ligaturés ensemble avec le plasmide pMB016 (voir exemple 6), préalablement digéré par EcoRI et Sall, pour donner le plasmide pEL044 de 7639 pb (figure 29). Ce plasmide permet l'insertion de la cassette d'expression HCMV-IE/NDV F dans le site UL43 du virus HVT.
Une co-transfection réalisée, comme décrit dans l'exemple 5, avec le plasmide pEL044 et l'ADN génomique du virus HVT a conduit à l'isolement et à la purification du recombinant vHVT9.

### Exemple 11: Construction du plasmide donneur pEL082 et isolement de vHVT10

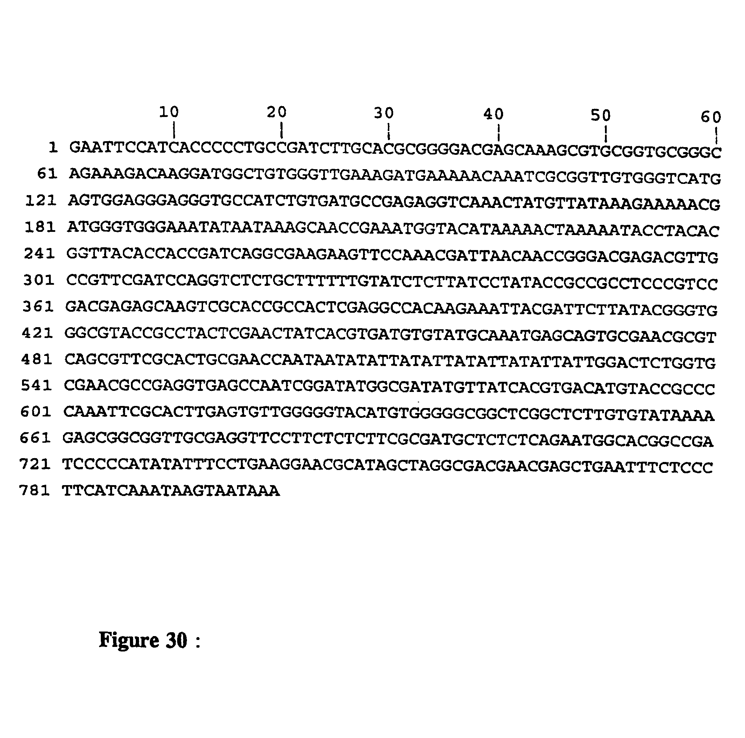

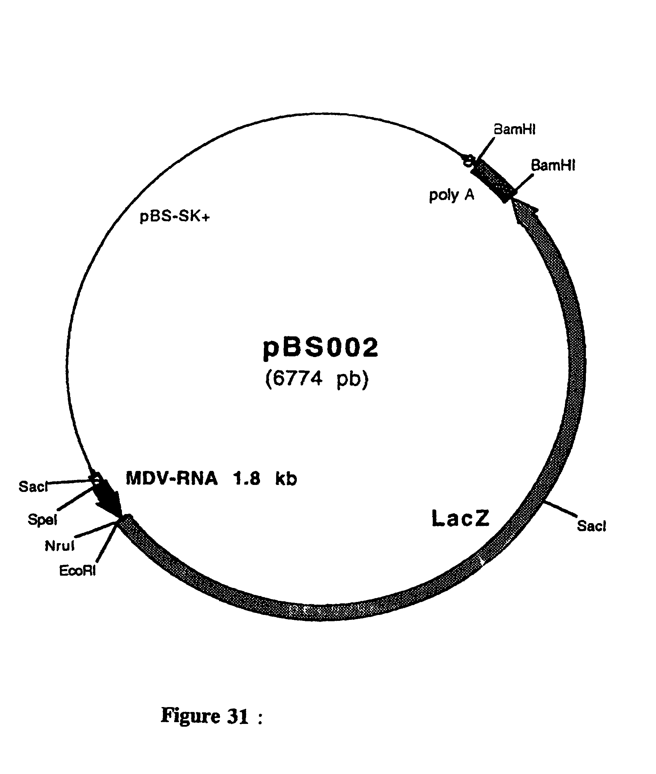

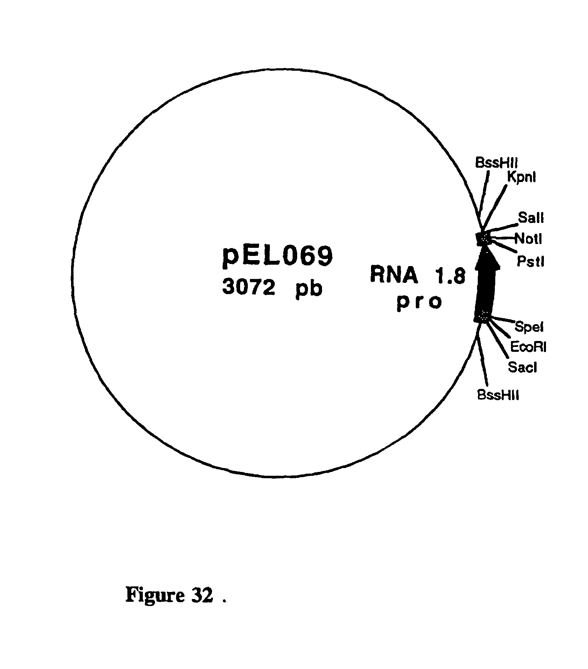

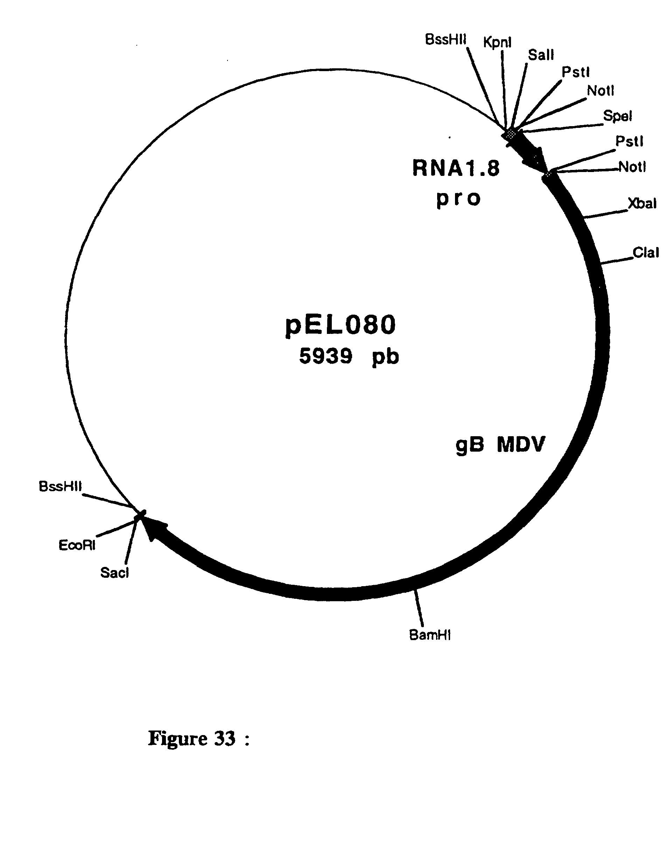

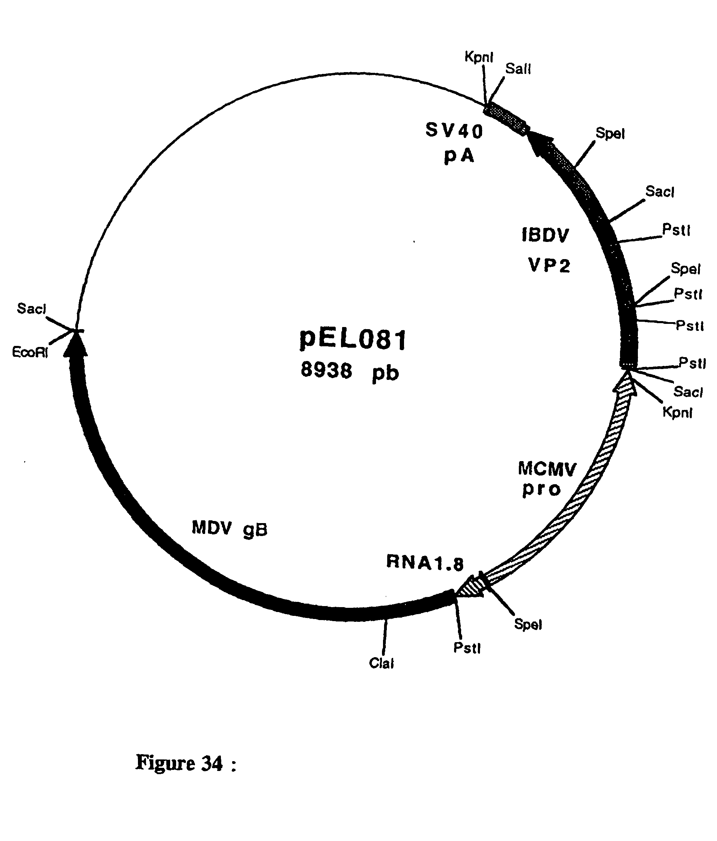

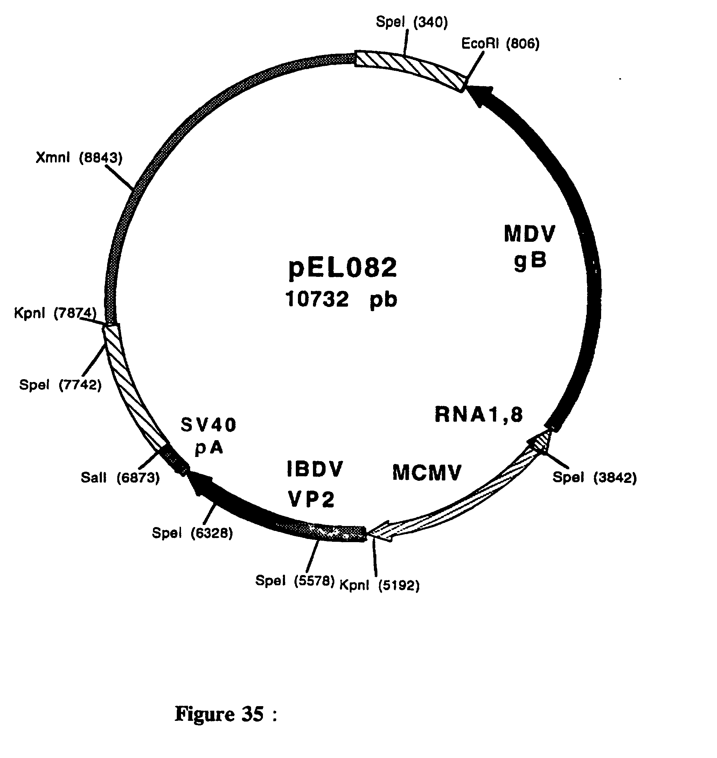

Les séquences situées en amont du gène MDV RNA 1.8 kpb sont décrites dans Bradley G. *et al*. (J. Virol. 1989. **63**. 2534-2542) (figure 30 et SEQ ID N° 22). Une amplification PCR a été réalisée à partir d'ADN extrait de lymphocytes récoltés sur des poulets infectés par la souche MDV RB1B (voir exemple 1) avec les oligonucléotides suivants: 
MB047 (SEQ ID N° 23) 5' GGTCTACTAGTATTGGACTCTGGTGCGAACGC 3'
MB048 (SEQ ID N° 24) 5' GTCCAGAATTCGCGAAGAGAGAAGGAACCTC 3'
 Le fragment PCR de 163 pb ainsi obtenu a été digéré par EcoRI et Spel, puis ligaturé avec le plasmide pCD002 (voir exemple 7), préalablement digéré par EcoRI et Spel, pour donner le plasmide pBS002 de 6774 pb (figure 31). Le plasmide pBS002 contient le promoteur du gène MDV RNA 1.8 kb cloné en amont du gène *lacZ*.
Une PCR a été réalisée avec les oligonucléotides: 
MB047 (SEQ ID N° 23) et
MB072 (SEQ ID N° 25)
5' GTGTCCTGCAGTCGCGAAGAGAGAAGGAACCTC 3'
 et la matrice pBS002. Le fragment PCR ainsi obtenu a été digéré par Pstl et Spel pour isoler un fragment Pstl-Spel de 200 pb. Ce fragment a été ligaturé avec le plasmide pEL067 (voir exemple 7), préalablement digéré par Pstl et Spel, pour donner le plasmide pEL069 (figure 32). Le plasmide pCD007 (voir exemple 8) a été digéré par EcoRI et Xbal pour isoler le fragment EcoRI-XbaI de 2670 pb (fragment A). Le plasmide pCD01 (voir exemple 8) a été digéré par Notl et Xbal pour isoler le fragment Notl-Xbal de 180 pb (fragment B). Le plasmide pEL069 a été digéré par Notl et Spel pour isoler le fragment Notl-Spel de 180 pb (fragment C). Les fragments A, B et C ont été ligaturés ensemble avec le plasmide pEL067 (voir exemple 7), préalablement digéré par EcoRI et Spel, pour donner le plasmide pEL080 de 5939 pb (figure 33). Le plasmide pEL070 (voir exemple 7) a été digéré par Kpnl et Spel pour isoler le fragment Kpnl-Spel de 1345 pb (fragment D). Le plasmide pEL070 a aussi été digéré par Kpnl et Sall pour isoler le fragment Kpnl-Sall de 1658 pb (fragment E). Les fragments D et E ont été ligaturés ensemble avec le plasmide pEL080, préalablement digéré par Sall et Spel, pour donner le plasmide pEL081 de 8938 pb (figure 34). Le plasmide pEL081 a été digéré par EcoRI et SalI pour isoler le fragment EcoRI-Sall de 6066 pb. Ce fragment a été ligaturé avec le plasmide pMB016 (voir exemple 6), préalablement digéré par EcoRI et Sall, pour donner finalement le plasmide pEL082 de 10732 pb (figure 35). Ce plasmide permet d'insérer la double cassette d'expression VP2/MCMV-IE//RNA 1.8 kpb/MDV gB dans le site UL43 du virus HVT.
Une co-transfection réalisée, comme décrit dans l'exemple 5, avec le plasmide pEL082 et l'ADN génomique du virus HVT a conduit à l'isolement et à la purification du recombinant vHVT10.

### Exemple 12: Construction du plasmide donneur pEL096 et isolement de vHVT22

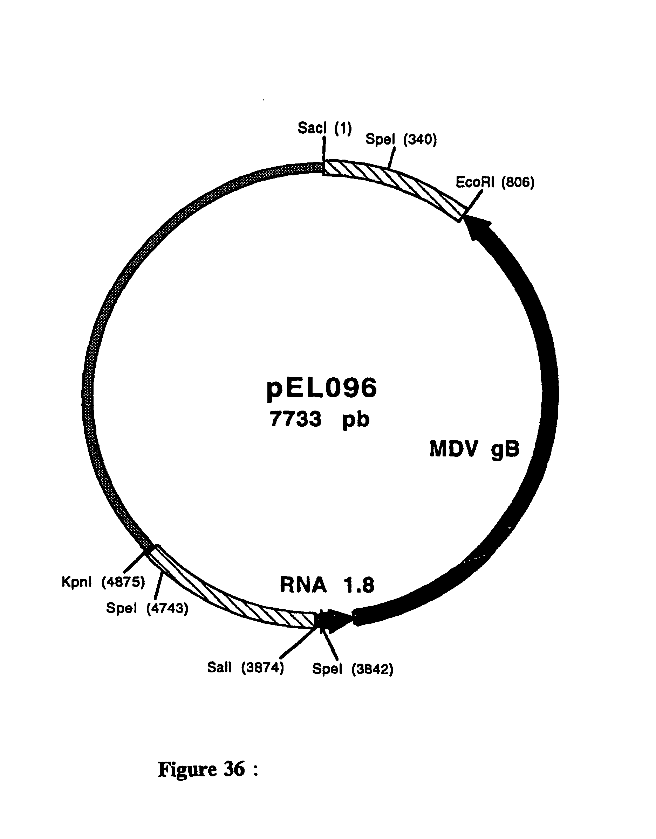

Le plasmide pEL080 (voir exemple 11) a été digéré par EcoRI et Sall pour isoler le fragment EcoRI-SalI de 3040 pb (cassette RNA 1.8 kpb / MDV gB). Ce fragment a été ligaturé avec le plasmide pMB016 (voir exemple 6), préalablement digéré par EcoRI et Sall, pour donner le plasmide pEL096 de 7733 pb (figure 36). Ce plasmide permet d'insérer la cassette d'expression RNA 1.8 kpb / MDV gB dans le site UL43 du virus HVT.
Une co-transfection réalisée, comme décrit dans l'exemple 5, avec le plasmide pEL096 et l'ADN génomique du virus HVT a conduit à l'isolement et à la purification du recombinant vHVT22.

### Exemple 13 : Construction de plasmides donneurs pour l'insertion de cassettes d'expression IBV M et S dans le site UL43 du virus HVT.

Selon la même stratégie que celle décrite plus haut pour l'insertion de cassettes d'expression (gènes placés sous le contrôle des promoteurs HCMV-IE ou MCMV-IE ou double promoteur MCMV-IE/RNA 1.8 kpb) dans le site UL43, il est possible de réaliser des virus HVT recombinants exprimant à un niveau élevé les protéines membrane (M) ou spike (S) du virus de la bronchite infectieuse aviaire (IBV). On réalise de préférence une construction où le gène IBV S est sous la dépendance du promoteur HCMV-IE ou du promoteur MCMV-IE, ou bien une construction où les gènes IBV M et IBV S sont insérés ensemble avec le double promoteur MCMV-IE/RNA 1.8 kpb dans le site UL43, le gène M étant sous le contrôle du promoteur RNA 1.8 kpb et le gène S étant sous le contrôle du promoteur MCMV-IE. Dans cette configuration, le promoteur RNA 1.8 kpb est activé par la région activatrice du promoteur MCMV-IE.

### Exemple 14 : Préparation d'un vaccin selon l'invention :

La préparation des vaccins selon l'invention peut se faire par toute technique usuelle connue de l'homme de l'art, par exemple par culture en flacon roulant. Des flacons roulants (175 cm²), ensemencés avec 200.10⁶ cellules primaires d'embryon de poulet, sont inoculés après 24 heures d'incubation à 37°C avec 1 ml d'une solution virale de virus HVT recombinant ayant un titre de 10⁵ pfu/ml. Après une incubation de 4 jours à 37°C, le surnageant est éliminé, les cellules sont dissociées avec une solution de trypsine versène, puis récoltées. Les cellules infectées sont alors centrifugées. Le surnageant est éliminé et les cellules sont reprises par 20 ml d'une solution contenant un stabilisateur pour lyophilisation (par exemple SPGA saccharose, phosphate, glutamate, albumine). Ce mélange est ensuite soniqué, réparti dans des flacons à raison de fractions de 1 ml et enfin lyophilisé.

Si nécessaire, le vaccin peut également être réparti et congelé au lieu d'être lyophilisé.

### Conclusion :

Des essais de protection par vaccination de poulets avec les différents virus HVT recombinants selon l'invention exprimant le gène IBDV VP2 ont montré que ces recombinants étaient capables d'induire un excellent niveau de protection contre une épreuve virulente IBDV.

## Revendications

1. Vaccin vivant recombinant aviaire comprenant, comme vecteur, un virus de la maladie de Marek (MDV ou HVT) comprenant au moins une séquence nucléotidique codant pour, et exprimant, un polypeptide antigénique d'un agent pathogène aviaire, insérée dans l'ORF du virus de la maladie de Marek codant pour une protéine homologue du produit du gène HSV-1 UL43 (gène UL43) sous le contrôle du promoteur CMV immediate early.

2. Vaccin vivant recombinant aviaire selon la revendication 1, **caractérisé en ce que** la séquence nucléotidique est insérée dans le gène UL43 après délétion totale ou partielle de ce gène.

3. Vaccin vivant recombinant aviaire selon l'une quelconque des revendications 1 et 2, **caractérisé en ce que** le promoteur CMV immediate early est le promoteur humain HCMV lE ou le promoteur murin MCMV IE.

4. Vaccin vivant recombinant aviaire, selon l'une quelconque des revendications 1 à 3, **caractérisé en ce que** la séquence nucléotidique insérée dans le gène UL43 sous le contrôle du promoteur CMV immediate early est une séquence nucléotidique codant pour un antigène choisi parmi le groupe des antigènes du virus de la maladie de Gumboro, du virus de la maladie de Marek, du virus de la maladie de Newcastie, du virus de la bronchite infectieuse, du virus de la laryngotrachéite infectieuse et du virus de l'anémie aviaire.

5. Vaccin vivant recombinant aviaire selon l'une quelconque des revendications 1 à 4, **caractérisé en ce que** la séquence nucléotidique insérée dans le gène UL43 sous le contrôle du promoteur CMV immediate early est une séquence nucléotidique codant pour le polypeptide VP2 du virus IBDV.

6. Vaccin vivant recombinant aviaire selon l'une quelconque des revendications 1 à 5, **caractérisé en ce qu'**il comprend, associé au promoteur CMV immediate early en disposition tête-bêche avec ce dernier, un autre promoteur, deux séquences nucléotidiques étant insérées dans le gène UL43, l'une sous la dépendance du promoteur CMV immediate early, l'autre sous celle du promoteur associé.

7. Vaccin vivant recombinant aviaire selon la revendication 6, **caractérisé en ce que** le promoteur associé est le promoteur Marek RNA1.8.

8. Vaccin vivant recombinant aviaire selon la revendication 6 ou 7, **caractérisé en ce que** la séquence nucléotidique insérée sous le contrôle du promoteur CMV immediate early est une séquence nucléotidique codant pour le polypeptide VP2 du virus IBDV et **en ce que** la séquence nucléotidique insérée sous le contrôle du promoteur associé est une séquence nucléotidique codant pour un antigène d'un autre pathogène aviaire.

9. Vaccin vivant recombinant aviaire selon la revendication 8, **caractérisé en ce que** l'antigène d'un autre pathogène aviaire est choisi parmi le groupe des antigènes du virus de la maladie de Marek, du virus de la maladie de Newcastle, du virus de la bronchite infectieuse, du virus de la laryngotrachéite infectieuse et du virus de l'anémie aviaire.

10. Vaccin vivant recombinant aviaire selon la revendication 6, **caractérisé en ce que** le promoteur associé est un promoteur CMV immediate. early d'origine différente.

11. Vaccin vivant recombinant aviaire selon l'une quelconque des revendications 1 à 10, **caractérisé en ce que** la ou les séquences nucléotidiques insérées dans le gène UL43 sont choisies parmi le groupe des séquences codant pour les gènes :
- VP2, VP3 et VP2 + VP4 + VP3 du virus de la maladie de Gumboro,
- gB, gC, gD et gH + gL des virus de la maladie de Marek,
- VP1 (52 kDa) + VP2 (24 kDa) du virus de l'anémie aviaire,
- S et M du virus de la bronchite infectieuse, et
- gB, gC, gD et gH + gL du virus de la laryngotrachéite infectieuse.

12. Formule de vaccin multivalent pour la vaccination contre plusieurs pathogènes aviaires, comprenant, en mélange ou à mélanger, au moins deux vaccins vivants recombinants aviaires tels que définis dans l'une quelconque des revendications 1 à 11, ces vaccins comprenant des séquences insérées différentes.

13. Formule de vaccin multivalent selon la revendication 12, **caractérisée en ce que** les séquences nucléotidiques insérées différentes proviennent de pathogènes différents.

14. Vaccin ou formule de vaccin multivalent selon l'une quelconque des revendications 1 à 13, **caractérisé en ce que** le vecteur est MDV.

15. Vaccin ou formule de vaccin multivalent selon l'une quelconque des revendications 1 à 13, **caractérisé en ce que** le vecteur est HVT.

## Patentansprüche

1. Rekombinanter Vogel-Lebendimpfstoff umfassend als Vektor ein Virus der Marek-Krankheit (MDV oder HVT), das mindestens eine Nukleotidsequenz umfaßt, die für ein antigenes Polypeptid eines pathogenen Agens aus einem Vogel kodiert und dieses exprimiert und unter der Kontrolle des Immediate-early-Promotors CMV in den ORF des Virus der Marek-Krankheit inseriert ist (Gen UL43) und für ein zu dem Produkt des Gens HSV-1 UL43 homologes Protein kodiert.

2. Rekombinanter Vogel-Lebendimpfstoff gemäß Anspruch 1, **dadurch gekennzeichnet, daß** die Nukleotidsequenz nach der vollständigen oder teilweisen Deletion des Gens UL43 in dieses Gen inseriert ist.

3. Rekombinanter Vogel-Lebendimpfstoff gemäß einem der Ansprüche 1 und 2, **dadurch gekennzeichnet, daß** der Immediate-early-Promotor CMV der humane Promotor HCMV IE oder der murine Promotor MCMV IE ist.

4. Rekombinanter Vogel-Lebendimpfstoff gemäß einem der Ansprüche 1 bis 3, **dadurch gekennzeichnet, daß** die unter der Kontrolle des Immediate-early-Promotors CMV inserierte Nukleotidsequenz eine Nukleotidsequenz ist, die für ein Antigen kodiert, das aus der Gruppe der Antigene des Virus der Gumboro-Krankheit, des Virus der Marek-Krankheit, des Virus der Newcastle-Krankheit, des Virus der infektiösen Bronchitis, des Virus der infektiösen Laryngotracheitis und des Virus der Vogelanämie ausgewählt ist.

5. Rekombinanter Vogel-Lebendimpfstoff gemäß einem der Ansprüche 1 bis 4, **dadurch gekennzeichnet, daß** die unter der Kontrolle des Immediate-early-Promotors CMV in das Gen UL43 inserierte Nukleotidsequenz eine Nukleotidsequenz ist, die für das Polypeptid VP2 des IBDV-Virus kodiert.

6. Rekombinanter Vogel-Lebendimpfstoff gemäß einem der Ansprüche 1 bis 5, **dadurch gekennzeichnet, daß** er einen weiteren Promotor, der mit dem Immediate-early-Promotor CMV verbunden ist und dazu entgegengesetzt angeordnet ist, umfaßt, bei dem zwei Nukleotidsequenzen in das Gen UL43 inseriert sind, wovon sich eine in Abhängigkeit von dem Immediate-early-Promotor CMV und die andere von dem damit verbundenen Promotor befindet.

7. Rekombinanter Vogel-Lebendimpfstoff gemäß Anspruch 6, **dadurch gekennzeichnet, daß** der damit verbundene Promotor der Promotor Marek RNA 1.8 ist.

8. Rekombinanter Vogel-Lebendimpfstoff gemäß Anspruch 6 oder 7, **dadurch gekennzeichnet, daß** die unter Kontrolle des Immediate-early-Promotors CMV inserierte Nukleotidsequenz eine Nukleotidsequenz ist, die für das Polypeptid VP2 des Virus IBDV kodiert und dadurch, daß die unter der Kontrolle des damit verbundenen Promotors inserierte Nukleotidsequenz eine Nukleotidsequenz ist, die für ein Antigen eines anderen Vogel-Pathogens kodiert.

9. Rekombinanter Vogel-Lebendimpfstoff gemäß Anspruch 8, **dadurch gekennzeichnet, daß** das Antigen eines anderen Vogel-Pathogens aus der Gruppe der Antigene des Virus der Marek-Krankheit, des Virus der Newcastle-Krankheit, des Virus der infektiösen Bronchitis, des Virus der Laryngotracheitis und des Virus der Vogelanämie ausgewählt ist.

10. Rekombinanter Vogel-Lebendimpfstoff gemäß Anspruch 6, **dadurch gekennzeichnet, daß** der damit verbundene Promotor ein Immediate-early-Promotor CMV von unterschiedlicher Herkunft ist.

11. Rekombinanter Vogel-Lebendimpfstoff gemäß einem der Ansprüche 1 bis 10, **dadurch gekennzeichnet, daß** die in das Gen UL43 inserierte(n) Nukleotidsequenz(en) aus der Gruppe der Sequenzen ausgewählt ist (sind), die für die Gene:
- VP2, VP3 und VP2+VP4+VP3 des Virus der Gumboro-Krankheit,
- gB, gC, gD und gH+gL des Virus der Marek-Krankheit,
- VP1 (52 kDa) + VP2 (24 kDa) des Virus der Vogelanämie,
- S und M des Virus der infektiösen Bronchitis und
- gB, gC, gD und gH+gL des Virus der infektiösen Laryngotracheitis kodieren.

12. Formulierung eines multivalenten Impfstoffs zur Impfung gegen mehrere Vogelantigene umfassend im Gemisch oder zum Mischen mindestens zwei in einem der Ansprüche 1 bis 11 definierte rekombinante Vogel-Lebendimpfstoffe, wobei diese Impfstoffe unterschiedliche inserierte Sequenzen umfassen.

13. Formulierung eines multivalenten Impfstoffs gemäß Anspruch 12, **dadurch gekennzeichnet, daß** die unterschiedlichen inserierten Nukleotidsequenzen aus unterschiedlichen Pathogenen stammen.

14. Impfstoff oder Formulierung eines multivalenten Impfstoffs gemäß einem der Ansprüche 1 bis 13, **dadurch gekennzeichnet, daß** der Vektor MDV ist.

15. Impfstoff oder Formulierung eines multivalenten Impfstoffs gemäß einem der Ansprüche 1 bis 13, **dadurch gekennzeichnet, daß** der Vektor HVT ist.

## Claims

1. A live recombinant avian vaccine comprising, as a vector, a Marek disease virus (MDV or HVT) comprising at least one nucleotide sequence encoding and expressing an antigenic polypeptide of an avian pathogenic agent, inserted into the ORF of the Marek disease virus encoding a protein homologous to the product of the HSV-1 UL43 gene (UL43 gene) under the control of the immediate early CMV promoter.

2. A live recombinant avian vaccine according to claim 1, **characterized in that** the nucleotide sequence is inserted into the UL43 gene after total or partial deletion of said gene.

3. A live recombinant avian vaccine according to claim 1 or claim 2, **characterized in that** the immediate early CMV promoter is the human HCMV IE promoter or the murine MCMV IE promoter.

4. A live recombinant avian vaccine according to any one of claims 1 to 3, **characterized in that** the nucleotide sequence inserted into the UL43 gene under the control of the immediate early CMV promoter is a nucleotide sequence coding for an antigen selected from the group of the antigens of the infectious bursal disease virus, the Marek disease virus, the Newcastle disease virus, the infectious bronchitis virus, the infectious laryngotracheitis virus and the avian anaemia virus.

5. A live recombinant avian vaccine according to any one of claims 1 to 4, **characterized in that** the nucleotide sequence inserted into the UL43 gene under the control of the immediate early CMV promoter is a nucleotide sequence coding for the VP2 polypeptide of the IBDV virus.

6. A live recombinant avian vaccine according to any one of claims 1 to 5, **characterized in that** it comprises a further promoter associated with the immediate early CMV promoter located head-to-tail thereto, two nucleotide sequences being inserted into the UL43 gene, one under the control of the immediate early CMV promoter, and the other under that of the associated promoter.

7. A live recombinant avian vaccine according to claim 6, **characterized in that** the associated promoter is the Marek RNA 1.8 promoter.

8. A live recombinant avian vaccine according to claim 6 or claim 7, **characterized in that** the nucleotide sequence inserted under the control of the immediate early CMV promoter is a nucleotide sequence coding for the VP2 polypeptide of the IBDV virus and **in that** the nucleotide sequence inserted under the control of the associated promoter is a nucleotide sequence coding for an antigen of another avian pathogen.

9. A live recombinant avian vaccine according to claim 8, **characterized in that** the antigen of another avian pathogen is selected from the group of the antigens of the Marek disease virus, the Newcastle disease virus, the infectious bronchitis virus, the infectious laryngotracheitis virus and the avian anaemia virus.

10. A live recombinant avian vaccine according to claim 6, **characterized in that** the associated promoter is an immediate early CMV promoter of different origin.

11. A live recombinant avian vaccine according to any one of claims 1 to 10, **characterized in that** the nucleotide sequence or sequences inserted into the UL43 gene is/are selected from the group of sequences coding for the following genes:
• VP2, VP3 and VP2+VP4+VP3 of infectious bursal disease virus;
• gB, gC, gD and gH+gL of Marek disease viruses;
• VP1 (52 kDa) + VP2 (24 kDa) of the avian anaemia virus;
• S and M of the infectious bronchitis virus; and
• gB, gC, gD and gH + gL of the infectious laryngotracheitis virus.

12. A multivalent vaccine formula for vaccination against a plurality of avian pathogens comprising, as a mixture or for mixing, at least two live recombinant avian vaccines as defined in any one of claims 1 to 11, said vaccines comprising different inserted sequences.

13. A multivalent vaccine formula according to claim 12, **characterized in that** the different inserted nucleotide sequences derive from different pathogens.

14. A vaccine or multivalent vaccine formula according to any one of claims 1 to 13, **characterized in that** the vector is MDV.

15. A vaccine or multivalent vaccine formula according to any one of claims 1 to 13, **characterized in that** the vector is HVT.
